# EUROPEAN PATENT APPLICATION

(11) **EP 1 914 221 A1**
(43) Date of publication of application: **23.04.2008**
(21) Application number: 06781393.1
(22) Date of filing: 14.07.2006
(51) Int. Cl.: C07C 209/68, C07C 211/27, C07C 211/45, C07C 211/52, C07C 221/00, C07C 225/22, C07B 61/00

(54) **PROCESS FOR PRODUCING BIARYL COMPOUND**

(30) Priority: 20.07.2005 JP 2005209531
(71) Applicant: Sumitomo Chemical Company, Limited, Tokyo 104-8260 (JP)
(72) Inventor: SATO, Koichi, Osaka 5670841 (JP); MIKI, Takashi, Osaka 5610802 (JP); UEDA, Hiroshi, Osaka 5610802 (JP)
(74) Representative: Cresswell, Thomas Anthony
(86) International application number: PCT/JP2006/314438
(87) International publication number: WO 2007/011005

(57) **Abstract**

A method for producing a biaryl compound, comprising reacting an aromatic organic compound with at least one compound selected from the group consisting of aromatic organoboron compounds and boroxine compounds, in the presence of a zero-valent nickel catalyst, phosphine ligand and base.

## Description

### Technical Field

The present invention relates to a method for producing a biaryl compound having an amino group.

### Background Art

As a method for producing a biaryl compound, there is known, for example, a method of coupling reaction of an aromatic organoboron compound with an aromatic organic compound such as aryl halides, in the presence of a palladium catalyst or nickel catalyst and a phosphine ligand, however, when a compound having an amino group is used as a raw material compound, catalytic activity lowers. As a method of improving catalytic activity in such a coupling reaction, there are variously developed methods using a di-valent nickel catalyst (see, Japanese Patent Application Laid-Open (JP-A) No. 2000-302697, J. Org. Chem., 62, 8024(1997)).

However, these methods are not necessarily industrially satisfactory methods because of necessity of performance of complicated catalyst pre-treatments, insufficient yield, and the like.

The present inventors have intensively investigated a method for more simply producing a biaryl compound having an amino group, and resultantly found that when a coupling reaction is carried out using a zero-valent nickel catalyst, phosphine ligand and base, the reaction progresses efficiently even if a raw material compound having an amino group is used, and the intended biaryl compound can be produced simply.

### DISCLOSURE OF THE INVENTION

The present invention has an object of producing a biaryl compound having an amino group, simply and efficiently.

That is, the present invention provides the following [1] to [10].
[1] A method for producing a biaryl compound of the following formula (4), comprising reacting an aromatic organic compound of the following formula (1) with at least one compound selected from the group consisting of aromatic organoboron compounds of the following formula (2) and boroxine compounds of the following formula (3), in the presence of a zero-valent nickel catalyst, phosphine ligand and base: 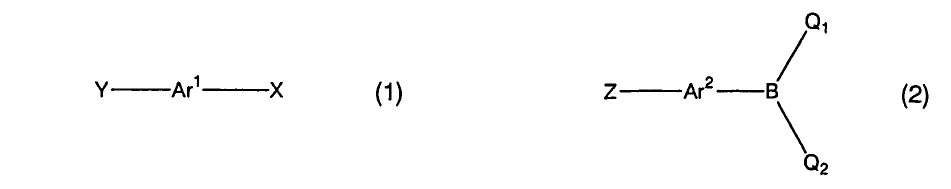 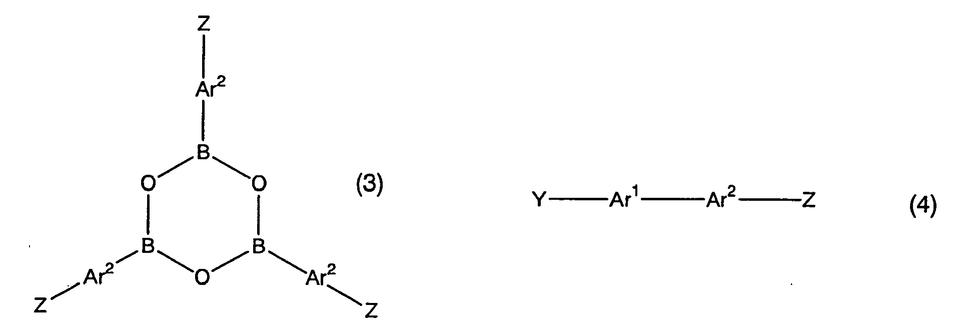 (wherein, Ar¹ and Ar² represent each independently an arylene group or heteroarylene group, the arylene group or heteroarylene group may have at least one substituent selected from the group consisting of optionally substituted alkyl groups, optionally substituted cycloalkyl groups, optionally substituted aryl groups, optionally substituted heteroaryl groups, optionally substituted alkoxy groups, optionally substituted aryloxy groups, optionally substituted alkylthio groups, optionally substituted arylthio groups, optionally substituted alkylcarbonyl groups, optionally substituted arylcarbonyl groups, optionally substituted alkylamino groups, optionally substituted alkylcarbonylamino groups, optionally substituted alkoxycarbonylamino groups, optionally substituted alkanesulfoneamide groups, optionally substituted arenesulfoneamide groups, optionally substituted N-arylcarbamoyl groups, optionally substituted imide groups, fluorine atom, hydroxyl group, formyl group, amino group, carbamoyl group, sulfamoyl group, mercapto group and imino group, substituents on adjacent carbon atoms on the arylene group or heteroarylene group may be mutually connected to form a condensed ring together with the arylene group or heteroarylene group,
   Q₁ and Q₂ represent each independently a hydroxyl group or an alkoxy group having a carbon number of 1 to 4, or Q₁ and Q₂ are connected to represent an alkylenedioxy group having a carbon number of 1 to 6 or a catecholate group,
   X represents a halogen atom, alkylsulfonyloxy group or arylsulfonyloxy group,
   Y and Z represent each independently a hydrogen atom, amino group or aminoalkyl group, Y and Z do not simultaneously represent a hydrogen atom, and a hydrogen atom in the alkyl of said aminoalkyl group may be substituted by a carboxyl group or alkoxycarbonyl group.).
[2] The production method according to [1], wherein the reaction is carried out in the presence of an ether solvent.
[3] The production method according to [1] or [2], wherein the zero-valent nickel catalyst is tetrakis(triphenyl phosphine)nickel(0), tetracarbonylnickel(0) or bis(1,5-cyclooctadiene)nickel(0).
[4] The production method according to any one of [1] to [3], wherein the zero-valent nickel catalyst is bis(1,5-cycloctadiene)nickel(0).
[5] The production method according to any one of [1] to [4], wherein the phosphine ligand is a trialkyl phosphine, tricycloalkyl phosphine or triaryl phosphine.
[6] The production method according to any one of [1] to [5], wherein the phosphine ligand is a tricycloalkyl phosphine.
[7] The production method according to any one of [1] to [6], wherein the base is at least one selected from the group consisting of alkali metal hydroxides, alkaline earth metal hydroxides, alkali metal carbonates, alkaline earth metal carbonates, alkali metal carboxylates, alkaline earth metal carboxylates, alkali metal hydrogencarbonates, alkaline earth metal hydrogencarbonates, alkali metal phosphates, alkali metal fluorides and tertiary amines, or its hydrate.
[8] The production method according to any one of [1] to [7], wherein the base is an alkali metal phosphates.
[9] The production method according to any one of [1] to [8], wherein X in the formula (1) is a halogen atom.
[10] The production method according to [9], wherein the halogen atoms is a chlorine atom.

### MODES FOR CARRYING OUT THE INVENTION

The present invention will be illustrated in detail below.

In the above-described aromatic compounds of the formula (1) (hereinafter, referred to as aromatic organic compound (1)), organoboron compounds of the formula (2) (hereinafter, referred to as organoboron compound (2)) and boroxine compounds of the formula (3) (hereinafter, referred to as boroxine compound (3)), the arylene group among groups represented by Ar¹ and Ar² means a di-valent hydrocarbon aromatic group having a carbon number of 6 to 14, and examples thereof include a phenylene group, naphthylene group, anthrylene group, phenanthrylene group, indenylene group, fluorenylene group and the like. The heteroarylene group means a di-valent hetero aromatic group having a carbon number of 4 to 12, and examples thereof include a pyridinediyl group, furandiyl group, thiophenediyl group, pyrrolediyl group, imidazolediyl group and the like.

As the substituent which may be carried on the arylene group or heteroarylene group, at least one substituent is mentioned selected from the group consisting of optionally substituted alkyl groups, optionally substituted cycloalkyl groups, optionally substituted aryl groups, optionally substituted heteroaryl groups, optionally substituted alkoxy groups, optionally substituted aryloxy groups, optionally substituted alkylthio groups, optionally substituted arylthio groups, optionally substituted alkylcarbonyl groups, optionally substituted arylcarbonyl groups, optionally substituted alkylamino groups, optionally substituted alkylcarbonylamino groups, optionally substituted alkoxycarbonylamino groups, optionally substituted alkanesulfoneamide groups, optionally substituted arenesulfoneamide groups, optionally substituted N-arylcarbamoyl groups, optionally substituted imide groups, fluorine atom, hydroxyl group, formyl group, amino group, carbamoyl group, sulfamoyl group, mercapto group and imino group.

Here, examples of the alkyl group include linear or branched alkyl groups having a carbon number of 1 to 4 such as a methyl group, ethyl group, n-propyl group, isopropyl group, n-butyl group, 1-methyl-propyl group, 2-methyl-propyl group and the like. Such alkyl groups may be substituted by a fluorine atom, hydroxyl group and the like, and examples of alkyl groups substituted by these groups include a trifluoromethyl group and the like, a hydroxymethyl group and the like.

Examples of the cycloalkyl group include cycloalkyl groups having a carbon number of 5 to 8 such as a cyclopentyl group, cyclohexyl group, cycloheptyl group, cyclooctyl group and the like. Such cycloalkyl groups may be substituted by a fluorine atom, hydroxyl group and the like, and examples of cycloalkyl groups substituted by these groups include a perfluoropentyl group and the like, a hydroxypentyl group and the like.

Examples of the aryl group include aryl groups having a carbon number of 6 to 10 such as a phenyl group, naphthyl group and the like. Such aryl groups may be substituted by a fluorine atom, hydroxyl group and the like, and examples of aryl groups substituted by these groups include a perfluorophenyl group and the like, a hydroxyphenyl group and the like.

Examples of the heteroaryl group include heteroaryl groups having a carbon number of 4 to 11 such as a pyridyl group, furyl group, thienyl group and the like. Such heteroaryl groups may be substituted by a fluorine atom, hydroxyl group and the like, and examples of heteroaryl groups substituted by these groups include a fluoropyridyl group and the like, a hydroxypyridyl group and the like.

Examples of the alkoxy group include linear or branched alkoxy groups having a carbon number of 1 to 4 such as a methoxy group, ethoxy group, t-butoxy group and the like. Such alkoxy groups may be substituted by a fluorine atom, hydroxyl group and the like, and examples of alkoxy groups substituted by these groups include a trifluoromethoxy group and the like, a hydroxymethoxy group and the like.

Examples of the aryloxy group include aryloxy groups having a carbon number of 6 to 10 such as a phenoxy group and the like. Such aryloxy groups may be substituted by a fluorine atom, hydroxyl group and the like, and examples of aryloxy groups substituted by these groups include a fluorophenoxy group and the like, a hydroxyphenoxy group and the like.

Examples of the alkylthio group include linear or branched alkylthio groups having a carbon number of 1 to 4 such as a methylthio group, ethylthio group and the like. Such alkylthio groups may be substituted by a fluorine atom, hydroxyl group and the like, and examples of alkylthio groups substituted by these groups include a fluoromethylthio group and the like, a hydroxymethylthio group and the like.

Examples of the arylthio group include arylthio groups having a carbon number of 6 to 10 such as a phenylthio group and the like. Such arylthio groups may be substituted by a fluorine atom, hydroxyl group and the like, and examples of arylthio groups substituted by these groups include a fluorophenylthio group and the like, a hydroxyphenylthio group and the like.

Examples of the alkylcarbonyl group include alkylcarbonyl groups having a carbon number of 2 to 5 such as an acetyl group and the like. Such alkylcarbonyl groups may be substituted by a fluorine atom, hydroxyl group and the like, and examples of alkylcarbonyl groups substituted by these groups include a perfluoroacetyl group and the like, a hydroxyacetyl group and the like.

Examples of the arylcarbonyl group include arylcarbonyl groups having a carbon number of 7 to 11 such as a benzoyl group and the like. Such arylcarbonyl groups may be substituted by a fluorine atom, hydroxyl group and the like, and examples of arylcarbonyl groups substituted by these groups include a fluorophenylcarbonyl group and the like, a hydroxyphenylcarbonyl group and the like.

Examples of the alkylamino group include alkylamino groups having a carbon number of 1 to 6 such as a methylamino group, dimethylamino group, cyclohexylamino group, ethylamino group and the like. Such alkylamino groups may be substituted by a fluorine atom, hydroxyl group and the like, and examples of alkylamino groups substituted by these groups include a trifluoromethylamino group and the like, a hydroxymethylamino group and the like.

Examples of the alkylcarbonylamino group include alkylcarbonylamino groups having a carbon number of 2 to 5 such as an acetylamino and the like. Such alkylcarbonylamino groups may be substituted by a fluorine atom, hydroxyl group and the like, and examples of alkylcarbonylamino groups substituted by these groups include a trifluoroacetylamino group and the like, a hydroxyacetylamino group and the like.

Examples of the alkoxycarbonylamino group include alkoxycarbonylamino groups having a carbon number of 2 to 5 such as a methoxycarbonylamino group, t-butoxycarbonylamino group and the like. Such alkoxycarbonylamino groups may be substituted by a fluorine atom, hydroxyl group and the like, and examples of alkoxycarbonylamino groups substituted by these groups include a fluoromethoxycarbonylamino group and the like, a hydroxymethoxycarbonylamino group and the like.

Examples of the alkanesulfoneamide group include alkanesulfoneamide groups having a carbon number of 1 to 4 such as a methanesulfoneamide group and the like. Such alkanesulfoneamide groups may be substituted by a fluorine atom, hydroxyl group and the like, and examples of alkanesulfoneamide groups substituted by these groups include a trifluoromethanesulfoneamide group and the like, a hydroxymethanesulfoneamide group and the like.

Examples of the arenesulfoneamide group include arenesulfoneamide groups having a carbon number of 6 to 10 such as a benzenesulfoneamide group, toluenesulfoneamide group and the like. Such arenesulfoneamide groups may be substituted by a fluorine atom, hydroxyl group and the like, and examples of arenesulfoneamide groups substituted by these groups include a pefluorophenylsulfoneamide group and the like, a hydroxyphenylsulfoneamide group and the like.

Examples of the N-arylcarbamonyl group include N-arylcarbamoyl groups having a carbon number of 7 to 11 such as a N-phenylcarbamoyl group and the like. Such N-arylcarbamoyl groups may be substituted by a fluorine atom, hydroxyl group and the like, and examples of N-arylcarbamoyl groups substituted by these groups include a N-(fluorophenyl)carbamoyl group and the like, a N-(hydroxyphenyl)carbamoyl group and the like.

Examples of the imide group include imide groups having a carbon number of 4 to 12 such as a succinimide group, phthalimide group and the like. Such imide groups may be substituted by a fluorine atom, hydroxyl group and the like, and examples of imide groups substituted by these groups include a fluorophthalimide group and the like, a hydroxyphthalimide group and the like.

Examples of the condensed ring to be formed together with the arylene group or heteroarylene group by mutual connection of substituents on adjacent carbon atoms on the arylene group or heteroarylene group include an indolediyl group and the like, a quinolinediyl group and the like.

Examples of the alkoxy group having a carbon number of 1 to 4, among substituents represented by Q₁ and Q₂, include a methoxy group, ethoxy group, n-propoxy group, isopropoxy group, t-butoxy group and the like. Examples of the alkylenedioxy group having a carbon number of 1 to 6 to be formed by connection of Q₁ and Q₂, include a pinacolate group, neopentyl glycolate group and the like.

X represents a halogen atom, alkylsulfonyloxy group or arylsulfonyloxy group. The halogen atom includes a chlorine atom, bromine atom, iodine atom and the like, and of them, a chlorine atom is preferable. Examples of the alkylsulfonyloxy group include a methanesulfonyloxy group, trifluoromethanesulfonyloxy group and the like, and examples of the arylsulfonyloxy group include a p-toluenesulfonyloxy group, p-trifluoromethanephenylsulfonyloxy group and the like.

Y and Z represent each independently a hydrogen atom, amino group or aminoalkyl group, Y and Z do not simultaneously represent a hydrogen atom, and a hydrogen atom in the alkyl of the above-described aminoalkyl group may be substituted by a carboxyl group or alkoxycarbonyl group.

The aminoalkyl group means an alkyl group having a carbon number of 1 to 5 substituted by an amino group, and examples thereof include an aminomethyl group, 1-aminoethyl group, 2-aminoethyl group, 1-aminopropyl group, 2-aminopropyl group, 3-aminopropyl group, 1-amino-1-methylethyl group, 2-amino-1-methylethyl group, 1-aminobutyl group, 2-aminobutyl group, 3-aminobutyl group, 4-aminobutyl group, 1-amino-1-methyl-butyl group, 2-amino-1-methyl-butyl group, 3-amino-1-methyl-butyl group, 1-amino-2-methyl-propyl group, 2-amino-2-methyl-propyl group, 3-amino-2-methyl-propyl group, 2-amino-1,1,-dimethyl-ethyl group and the like.

Specific examples of the organic aromatic compound (1) include the following compounds.
chlorobenzene, 2-chlorotoluene, 3-chlorotoluene, 4-chlorotoluene, 1-chloro-2,3-dimethylbenzene, 1-chloro-2,4-dimethylbenzene, 1-chloro-2,5-dimethylbenzene, 1-chloro-2,6-dimethylbenzene, 1-chloro-3,5-dimethylbenzene, 1-chloro-3,4-dimethylbenzene, 1-chloro-2-ethylbenzene, 1-chloro-3-ethylbenzene, 1-chloro-4-ethylbenzene, 1-chloro-2,3-diethylbenzene, 1-chloro-2,4-diethylbenzene, 1-chloro-2,5-diethylbenzene, 1-chloro-2,6-diethylbenzene, 1-chloro-3,5-diethylbenzene, 1-chloro-3,4-diethylbenzene, 1-chloro-2-ethyl-3-methylbenzene, 1-chloro-3-ethyl-2-methylbenzene, 1-chloro-2-ethyl-6-methylbenzene, 1-chloro-3-ethyl-5-methylbenzene, 1-chloro-4-propylbenzene, 1-chloro-4-isopropylbenzene, 1-chloro-2-propylbenzene, 1-chloro-2-isopropylbenzene, 1-chloro-3-propylbenzene, 1-chloro-3-isopropylbenzene, 4-butyl-1-chlorobenzene, 1-chloro-2-(1-methylpropyl)benzene, 1-chloro-3-(1-methylpropyl)benzene, 1-chloro-4-(1-methylpropyl)benzene, 1-chloro-2-(2-methylpropyl)benzene, -chloro-3-(2-methylpropyl)benzene, 1-chloro-4-(2-methylpropyl)benzene, 4-t-butyl-1-chlorobenzene,
2-chloroaniline, 3-chloroaniline, 4-chloroaniline, 2-chloro-3-methylaniline, 2-chloro-4-methylaniline, 2-chloro-5-methylaniline, 2-chloro-6-methylaniline, 3-chloro-2-methylaniline, 3-chloro-4-methylaniline, 3-chloro-5-methylaniline, 3-chloro-6-methylaniline, 4-chloro-2-methylaniline, 4-chloro-3-methylaniline, 2-chloro-3-ethylaniline, 2-chloro-4-ethylaniline, 2-chloro-5-ethylaniline, 2-chloro-6-ethylaniline, 3-chloro-2-ethylaniline, 3-chloro-4-ethylaniline, 3-chloro-5-ethylaniline, 3-chloro-6-ethylaniline, 4-chloro-2-ethylaniline, 4-chloro-3-ethylaniline,
2-chloro-3-propylaniline, 2-chloro-4-propylaniline, 2-chloro-5-propylaniline, 2-chloro-6-propylaniline, 3-chloro-2-propylaniline, 3-chloro-4-propylaniline, 3-chloro-5-propylaniline, 3-chloro-6-propylaniline, 4-chloro-2-propylaniline, 4-chloro-3-propylaniline, 2-chloro-3-isopropylaniline, 2-chloro-4-isopropylaniline, 2-chloro-5-isopropylaniline, 2-chloro-6-isopropylaniline, 3-chloro-2-isopropylaniline, 3-chloro-4-isopropylaniline, 3-chloro-5-isopropylaniline, 3-chloro-6-isopropylaniline, 4-chloro-2-isopropylaniline, 4-chloro-3-isopropylaniline, 2-chloro-3-butylaniline, 2-chloro-4-butylaniline, 2-chloro-5-butylaniline, 2-chloro-6-butylaniline, 3-chloro-2-butylaniline, 3-chloro-4-butylaniline, 3-chloro-5-butylaniline, 3-chloro-6-butylaniline, 4-chloro-2-butylaniline, 4-chloro-3-butylaniline, ,
2-chloro-3-t-butylaniline, 2-chloro-4-t-butylaniline, 2-chloro-5-t-butylaniline, 2-chloro-6-t-butylaniline, 3-chloro-2-t-butylaniline, 3-chloro-4-t-butylaniline, 3-chloro-5-t-butylaniline, 3-chloro-6-t-butylaniline, 4-chloro-2-t-butylaniline, 4-chloro-3-t-butylaniline,
1-chloro-2-fluorobenzene, 1-chloro-3-fluorobenzene, 1-chloro-4-fluorobenzene, 1-chloro-2,3-difluorobenzene, 1-chloro-2,4-difluorobenzene, 1-chloro-2,5-difluorobenzene, 1-chloro-2,6-difluorobenzene, 1-chloro-3,4-difluorobenzene, 1-chloro-3,5-difluorobenzene, 1-chloro-3,6-difluorobenzene, ,
2-chloro-3-fluoroaniline, 2-chloro-4-fluoroaniline, 2-chloro-5-fluoroaniline, 2-chloro-6-fluoroaniline, 3-chloro-2-fluoroaniline, 3-chloro-4-fluoroaniline, 3-chloro-5-fluoroaniline, 3-chloro-6-fluoroaniline, 4-chloro-2-fluoroaniline, 4-chloro-3-fluoroaniline,
1-chloro-2-(trifluoromethyl)benzene, 1-chloro-3-(trifluoromethyl)benzene, 1-chloro-4-(trifluoromethyl)benzene, 1-chloro-2,3-bis(trifluoromethyl)benzene, 1-chloro-2,4-bis(trifluoromethyl)benzene, 1-chloro-2,5-bis(trifluoromethyl)benzene, 1-chloro-2,6-bis(trifluoromethyl)benzene, 1-chloro-3,4-bis(trifluoromethyl)benzene, 1-chloro-3,5-bis(trifluoromethyl)benzene, 1-chloro-3,6-bis(trifluoromethyl)benzene,
2-chloro-3-(trifluoromethyl)aniline, 2-chloro-4-(trifluoromethyl)aniline, 2-chloro-5-(trifluoromethyl)aniline, 2-chloro-6-(trifluoromethyl)aniline, 3-chloro-2-(trifluoromethyl)aniline, 3-chloro-4-(trifluoromethyl)aniline, 3-chloro-5-(trifluoromethyl)aniline, 3-chloro-6-(trifluoromethyl)aniline, 4-chloro-2-(trifluoromethyl)aniline, 4-chloro-3-(trifluoromethyl)aniline,
1-chloro-2-cyclopropylbenzene, 1-chloro-3-cyclopropylbenzene, 1-chloro-4-cyclopropylbenzene, 1-chloro-2,3-dicyclopropylbenzene, 1-chloro-2,4-dicyclopropylbenzene, 1-chloro-2,5-dicyclopropylbenzene, 1-chloro-2,6-dicyclopropylbenzene, 1-chloro-3,4-dicyclopropylbenzene, 1-chloro-3,5-dicyclopropylbenzene, 1-chloro-3,6-dicyclopropylbenzene, 1-chloro-4,5-dicyclopropylbenzene, 1-chloro-4,6-dicyclopropylbenzene,
2-chloro-3-cyclopropylaniline, 2-chloro-4-cyclopropylaniline, 2-chloro-5-cyclopropylaniline, 2-chloro-6-cyclopropylaniline, 3-chloro-2-cyclopropylaniline, 3-chloro-4-cyclopropylaniline, 3-chloro-5-cyclopropylaniline, 3-chloro-6-cyclopropylaniline, 4-chloro-2-cyclopropylaniline, 4-chloro-3-cyclopropylaniline,
1-chloro-2-cyclobutylbenzene, 1-chloro-3-cyclobutylbenzene, 1-chloro-4-cyclobutylbenzene, 1-chloro-2,3-dicyclobutylbenzene, 1-chloro-2,4-dicyclobutylbenzene, 1-chloro-2,5-dicyclobutylbenzene, 1-chloro-2,6-dicyclobutylbenzene, 1-chloro-3,4-dicyclobutylbenzene, 1-chloro-3,5-dicyclobutylbenzene, 1-chloro-3,6-dicyclobutylbenzene,
2-chloro-3-cyclobutylaniline, 2-chloro-4-cyclobutylaniline, 2-chloro-5-cyclobutylaniline, 2-chloro-6-cyclobutylaniline, 3-chloro-2-cyclobutylaniline, 3-chloro-4-cyclobutylaniline, 3-chloro-5-cyclobutylaniline, 3-chloro-6-cyclobutylaniline, 4-chloro-2-cyclobutylaniline, 4-chloro-3-cyclobutylaniline,
1-chloro-2-cyclopentylbenzene, 1-chloro-3-cyclopentylbenzene, 1-chloro-4-cyclopentylbenzene, 1-chloro-2,3-dicyclopentylbenzene, 1-chloro-2,4-dicyclopentylbenzene, 1-chloro-2,5-dicyclopentylbenzene, 1-chloro-2,6-dicyclopentylbenzene, 1-chloro-3,4-dicyclopentylbenzene, 1-chloro-3,5-dicyclopentylbenzene, 1-chloro-3,6-dicyclopentylbenzene,
2-chloro-3-cyclopentylaniline, 2-chloro-4-cyclopentylaniline, 2-chloro-5-cyclopentylaniline, 2-chloro-6-cyclopentylaniline, 3-chloro-2-cyclopentylaniline, 3-chloro-4-cyclopentylaniline, 3-chloro-5-cyclopentylaniline, 3-chloro-6-cyclopentylaniline, 4-chloro-2-cyclopentylaniline, 4-chloro-3-cyclopentylaniline,
1-chloro-2-cyclohexylbenzene, 1-chloro-3-cyclohexylbenzene, 1-chloro-4-cyclohexylbenzene, 1-chloro-2,3-dicyclohexylbenzene, 1-chloro-2,4-dicyclohexylbenzene, 1-chloro-2,5-dicyclohexylbenzene, 1-chloro-2,6-dicyclohexylbenzene, 1-chloro-3,4-dicyclohexylbenzene, 1-chloro-3,5-dicyclohexylbenzene, 1-chloro-3,6-dicyclohexylbenzene,
2-chloro-3-cyclohexylaniline, 2-chloro-4-cyclohexylaniline, 2-chloro-5-cyclohexylaniline, 2-chloro-6-cyclohexylaniline, 3-chloro-2-cyclohexylaniline, 3-chloro-4-cyclohexylaniline, 3-chloro-5-cyclohexylaniline, 3-chloro-6-cyclohexylaniline, 4-chloro-2-cyclohexylaniline, 4-chloro-3-cyclohexylaniline,
2-chlorophenol, 3-chlorophenol, 4-chlorophenol, 1-chloro-2,3-dihydroxybenzene, 1-chloro-2,4-dihydroxybenzene, 1-chloro-2,5-dihydroxybenzene, 1-chloro-2,6-dihydroxybenzene, 1-chloro-3,4-dihydroxybenzene, 1-chloro-3,5-dihydroxybenzene,
2-amino-3-chlorophenol, 2-amino-4-chlorophenol, 2-amino-5-chlorophenol, 2-amino-6-chlorophenol, 3-amino-4-chlorophenol, 3-amino-5-chlorophenol, 3-amino-6-chlorophenol, 4-amino-3-chlorophenol,
2-chloro-5-methylphenol, 3-chloro-5-methylphenol, 4-chloro-5-methylphenol, 2-chloro-3,4-dihydroxytoluene, 2-chloro-3,5-dihydroxytoluene, 2-chloro-3,6-dihydroxytoluene, 3-chloro-2,4-dihydroxytoluene, 3-chloro-2,5-dihydroxytoluene, 3-chloro-2,6-dihydroxytoluene,
2-amino-3-chloro-4-methylphenol, 2-amino-4-chloro-5-methylphenol, 2-amino-5-chloro-6-methylphenol, 2-amino-6-chloro-3-methylphenol, 3-amino-4-chloro-5-methylphenol, 3-amino-5-chloro-6-methylphenol, 3-amino-6-chloro-2-methylphenol, 4-amino-3-chloro-5-methylphenol,
2-chloroanisole, 3-chloroanisole, 4-chloroanisole, 1-chloro-2,3-dimethoxybenzene, 1-chloro-2,4-dimethoxybenzene, 1-chloro-2,5-dimethoxybenzene, 1-chloro-2,6-dimethoxybenzene, 1-chloro-3,4-dimethoxybenzene, 1-chloro-3,5-dimethoxybenzene,
2-amino-3-chloroanisole, 2-amino-4-chloroanisole, 2-amino-5-chloroanisole, 2-amino-6-chloroanisole, 3-amino-2-chloroanisole, 3-amino-4-chloroanisole, 3-amino-5-chloroanisole, 3-amino-6-chloroanisole, 4-amino-2-chloroanisole, 4-amino-3-chloroanisole, 4-amino-5-chloroanisole, 4-amino-6-chloroanisole,
1-chloro-2-ethoxybenzene, 1-chloro-3-ethoxybenzene, 1-chloro-4-ethoxybenzene, 1-chloro-2,3-diethoxybenzene, 1-chloro-2,4-diethoxybenzene, 1-chloro-2,5-diethoxybenzene, 1-chloro-2,6-diethoxybenzene, 1-chloro-3,4-diethoxybenzene, 1-chloro-3,5-diethoxybenzene,
2-chloro-3-ethoxyaniline, 2-chloro-4-ethoxyaniline, 2-chloro-5-ethoxyaniline, 2-chloro-6-ethoxyaniline, 3-chloro-2-ethoxyaniline, 3-chloro-4-ethoxyaniline, 3-chloro-5-ethoxyaniline, 3-chloro-6-ethoxyaniline, 4-chloro-2-ethoxyaniline, 4-chloro-3-ethoxyaniline, 4-chloro-5-ethoxyaniline, 4-chloro-6-ethoxyaniline,
1-chloro-2-propoxybenzene, 1-chloro-3-propoxybenzene, 1-chloro-4-propoxybenzene, 1-chloro-2,3-dipropoxybenzene, 1-chloro-2,4-dipropoxybenzene, 1-chloro-2,5-dipropoxybenzene, 1-chloro-2,6-dipropoxybenzene, 1-chloro-3,4-dipropoxybenzene, 1-chloro-3,5-dipropoxybenzene,
2-chloro-3-propoxyaniline, 2-chloro-4-propoxyaniline, 2-chloro-5-propoxyaniline, 2-chloro-6-propoxyaniline, 3-chloro-2-propoxyaniline, 3-chloro-4-propoxyaniline, 3-chloro-5-propoxyaniline, 3-chloro-6-propoxyaniline, 4-chloro-2-propoxyaniline, 4-chloro-3-propoxyaniline, 4-chloro-5-propoxyaniline, 4-chloro-6-propoxyaniline,
1-chloro-2-butoxybenzene, 1-chloro-3-butoxybenzene, 1-chloro-4-butoxybenzene, 1-chloro-2,3-dibutoxybenzene, 1-chloro-2,4-dibutoxybenzene, 1-chloro-2,5-dibutoxybenzene, 1-chloro-2,6-dibutoxybenzene, 1-chloro-3,4-dibutoxybenzene, 1-chloro-3,5-dibutoxybenzene,
2-chloro-3-butoxyaniline, 2-chloro-4-butoxyaniline, 2-chloro-5-butoxyaniline, 2-chloro-6-butoxyaniline, 3-chloro-2-butoxyaniline, 3-chloro-4-butoxyaniline, 3-chloro-5-butoxyaniline, 3-chloro-6-butoxyaniline, 4-chloro-2-butoxyaniline, 4-chloro-3-butoxyaniline, 4-chloro-5-butoxyaniline, 4-chloro-6-butoxyaniline,
1-chloro-2-t-butoxybenzene, 1-chloro-3-t-butoxybenzene, 1-chloro-4-t-butoxybenzene, 1-chloro-2,3-di-t-butoxybenzene, 1-chloro-2,4-di-t-butoxybenzene, 1-chloro-2,5-di-t-butoxybenzene, 1-chloro-2,6-di-t-butoxybenzene, 1-chloro-3,4-di-t-butoxybenzene, 1-chloro-3,5-di-t-butoxybenzene,
2-chloro-3-t-butoxyaniline, 2-chloro-4-t-butoxyaniline, 2-chloro-5-t-butoxyaniline, 2-chloro-6-t-butoxyaniline, 3-chloro-2-t-butoxyaniline, 3-chloro-4-t-butoxyaniline, 3-chloro-5-t-butoxyaniline, 3-chloro-6-t-butoxyaniline, 4-chloro-2-t-butoxyaniline, 4-chloro-3-t-butoxyaniline, 4-chloro-5-t-butoxyaniline, 4-chloro-6-t-butoxyaniline,
1-chloro-2-phenoxybenzene, 1-chloro-3-phenoxybenzene, 1-chloro-4-phenoxybenzene, 1-chloro-2,3-diphenoxybenzene, 1-chloro-2,4-diphenoxybenzene, 1-chloro-2,5-diphenoxybenzene, 1-chloro-2,6-diphenoxybenzene, 1-chloro-3,4-diphenoxybenzene, 1-chloro-3,5-diphenoxybenzene,
2-chloro-3-phenoxyaniline, 2-chloro-4-phenoxyaniline, 2-chloro-5-phenoxyaniline, 2-chloro-6-phenoxyaniline, 3-chloro-2-phenoxyaniline, 3-chloro-4-phenoxyaniline, 3-chloro-5-phenoxyaniline, 3-chloro-6-phenoxyaniline, 4-chloro-2-phenoxyaniline, 4-chloro-3-phenoxyaniline, 4-chloro-5-phenoxyaniline, 4-chloro-6-phenoxyaniline,
2-chlorothiophenol, 3-chlorothiophenol, 4-chlorothiophenol, 1-chloro-2,3-dimercaptobenzene, 1-chloro-2,4-dimercaptobenzene, 1-chloro-2,5-dimercaptobenzene, 1-chloro-2,6-dimercaptobenzene, 1-chloro-3,4-dimercaptobenzene, 1-chloro-3,5-dimercaptobenzene,
2-amino-3-chlorothiophenol, 2-amino-4-chlorothiophenol, 2-amino-5-chlorothiophenol, 2-amino-6-chlorothiophenol, 3-amino-4-chlorothiophenol, 3-amino-5-chlorothiophenol, 3-amino-6-chlorothiophenol, 4-amino-3-chlorothiophenol,
2-chlorothioanisole, 3-chlorothioanisole, 4-chlorothioanisole, 1-chloro-2,3-(dimethylthio)benzene, 1-chloro-2,4-(dimethylthio)benzene, 1-chloro-2,5-(dimethylthio)benzene, 1-chloro-2,6-(dimethylthio)benzene, 1-chloro-3,4-(dimethylthio)benzene, 1-chloro-3,5-(dimethylthio)benzene,
2-amino-3-chlorothioanisole, 2-amino-4-chlorothioanisole, 2-amino-5-chlorothioanisole, 2-amino-6-chlorothioanisole, 3-amino-4-chlorothioanisole, 3-amino-5-chlorothioanisole, 3-amino-6-chlorothioanisole, 4-amino-3-chlorothioanisole,
1-chloro-2-ethylthiobenzene, 1-chloro-3-ethylthiobenzene, 1-chloro-4-ethylthiobenzene, 1-chloro-2,3-(diethylthio)benzene, 1-chloro-2,4-(diethylthio)benzene, 1-chloro-2,5-(diethylthio)benzene, 1-chloro-2,6-(diethylthio)benzene, 1-chloro-3,4-(diethylthio)benzene, 1-chloro-3,5-(diethylthio)benzene,
2-amino-3-chloro-1,-ethylthiobenzene, 2-amino-4-chloro-1,-ethylthiobenzene, 2-amino-5-chloro-1,-ethylthiobenzene, 2-amino-6-chloro-1,-ethylthiobenzene, 3-amino-4-chloro-1,-ethylthiobenzene, 3-amino-5-chloro-1,-ethylthiobenzene, 3-amino-6-chloro-1,-ethylthiobenzene, 4-amino-3-chloro-1,-ethylthiobenzene,
1-chloro-2-phenylthiobenzene, 1-chloro-3-phenylthiobenzene, 1-chloro-4-phenylthiobenzene, 1-chloro-2,3-(diphenylthio)benzene, 1-chloro-2,4-(diphenylthio)benzene, 1-chloro-2,5-(diphenylthio)benzene, 1-chloro-2,6-(diphenylthio)benzene, 1-chloro-3,4-(diphenylthio)benzene, 1-chloro-3,5-(diphenylthio)benzene,
2-amino-3-chloro-1,-phenylthiobenzene, 2-amino-4-chloro-1,-phenylthiobenzene, 2-amino-5-chloro-1,-phenylthiobenzene, 2-amino-6-chloro-1,-phenylthiobenzene, 3-amino-4-chloro-1,-phenylthiobenzene, 3-amino-5-chloro-1,-phenylthiobenzene, 3-amino-6-chloro-1,-phenylthiobenzene, 4-amino-3-chloro-1,-phenylthiobenzene,
1-chloro-2-(methylamino)benzene, 1-chloro-3-(methylamino)benzene, 1-chloro-4-(methylamino)benzene, 1-chloro-2,3-di(methylamino)benzene, 1-chloro-2,4-di(methylamino)benzene, 1-chloro-2,5-di(methylamino)benzene, 1-chloro-2,6-di(methylamino)benzene, 1-chloro-3,4-di(methylamino)benzene, 1-chloro-3,5-di(methylamino)benzene, 1-chloro-3,6-di(methylamino)benzene, 1-chloro-4,5-di(methylamino)benzene, 1-chloro-4,6-di(methylamino)benzene,
3-chloro-2-(methylamino)aniline, 4-chloro-2-(methylamino)aniline, 5-chloro-2-(methylamino)aniline, 6-chloro-2-(methylamino)aniline, 2-chloro-3-(methylamino)aniline, 4-chloro-3-(methylamino)aniline, 5-chloro-3-(methylamino)aniline, 6-chloro-3-(methylamino)aniline, 2-chloro-4-(methylamino)aniline, 3-chloro-4-(methylamino)aniline,
1-chloro-2-(ethylamino)benzene, 1-chloro-3-(ethylamino)benzene, 1-chloro-4-(ethylamino)benzene, 1-chloro-2,3-di(ethylamino)benzene, 1-chloro-2,4-di(ethylamino)benzene, 1-chloro-2,5-di(ethylamino)benzene, 1-chloro-2,6-di(ethylamino)benzene, 1-chloro-3,4-di(ethylamino)benzene, 1-chloro-3,5-di(ethylamino)benzene, 1-chloro-3,6-di(ethylamino)benzene, 1-chloro-4,5-di(ethylamino)benzene, 1-chloro-4,6-di(ethylamino)benzene,
3-chloro-2-(ethylamino)aniline, 4-chloro-2-(ethylamino)aniline, 5-chloro-2-(ethylamino)aniline, 6-chloro-2-(ethylamino)aniline, 2-chloro-3-(ethylamino)aniline, 4-chloro-3-(ethylamino)aniline, 5-chloro-3-(ethylamino)aniline, 6-chloro-3-(ethylamino)aniline, 2-chloro-4-(ethylamino)aniline, 3-chloro-4-(ethylamino)aniline,
1-chloro-2-(dimethylamino)benzene, 1-chloro-3-(dimethylamino)benzene, 1-chloro-4-(dimethylamino)benzene, 1-chloro-2,3-bis(dimethylamino)benzene, 1-chloro-2,4-bis(dimethylamino)benzene, 1-chloro-2,5-bis(dimethylamino)benzene, 1-chloro-2,6-bis(dimethylamino)benzene, 1-chloro-3,4-bis(dimethylamino)benzene, 1-chloro-3,5-bis(dimethylamino)benzene, 1-chloro-3,6-bis(dimethylamino)benzene, 1-chloro-4,5-bis(dimethylamino)benzene, 1-chloro-4,6-bis(dimethylamino)benzene,
3-chloro-2-(dimethylamino)aniline, 4-chloro-2-(dimethylamino)aniline, 5-chloro-2-(dimethylamino)aniline, 6-chloro-2-(dimethylamino)aniline, 2-chloro-3-(dimethylamino)aniline, 4-chloro-3-(dimethylamino)aniline, 5-chloro-3-(dimethylamino)aniline, 6-chloro-3-(dimethylamino)aniline, 2-chloro-4-(dimethylamino)aniline, 3-chloro-4-(dimethylamino)aniline,
1-chloro-2-(cyclohexylamino)benzene, 1-chloro-3-(cyclohexylamino)benzene, 1-chloro-4-(cyclohexylamino)benzene, 1-chloro-2,3-di(cyclohexylamino)benzene, 1-chloro-2,4-di(cyclohexylamino)benzene, 1-chloro-2,5-di(cyclohexylamino)benzene, 1-chloro-2,6-di(cyclohexylamino)benzene, 1-chloro-3,4-di(cyclohexylamino)benzene, 1-chloro-3,5-di(cyclohexylamino)benzene, 1-chloro-3,6-di(cyclohexylamino)benzene, 1-chloro-4,5-di(cyclohexylamino)benzene, 1-chloro-4,6-di(cyclohexylamino)benzene,
3-chloro-2-(cyclohexylamino)aniline, 4-chloro-2-(cyclohexylamino)aniline, 5-chloro-2-(cyclohexylamino)aniline, 6-chloro-2-(cyclohexylamino)aniline, 2-chloro-3-(cyclohexylamino)aniline, 4-chloro-3-(cyclohexylamino)aniline, 5-chloro-3-(cyclohexylamino)aniline, 6-chloro-3-(cyclohexylamino)aniline, 2-chloro-4-(cyclohexylamino)aniline, 3-chloro-4-(cyclohexylamino)aniline,
2-chloro-1-(methoxycarbonylamino)benzene, 3-chloro-1-(methoxycarbonylamino)benzene, 4-chloro-1-(methoxycarbonylamino)benzene, 3-chloro-1,2-di(methoxycarbonylamino)benzene, 4-chloro-1,2-di(methoxycarbonylamino)benzene, 2-chloro-1,3-di(methoxycarbonylamino)benzene, 4-chloro-1,3-di(methoxycarbonylamino)benzene, 5-chloro-1,3-di(methoxycarbonylamino)benzene, 2-chloro-1,4-di(methoxycarbonylamino)benzene, 3-chloro-1,4-di(methoxycarbonylamino)benzene,
2-chloro-1-(ethoxycarbonylamino)benzene, 3-chloro-1-(ethoxycarbonylamino)benzene, 4-chloro-1-(ethoxycarbonylamino)benzene, 3-chloro-1,2-di(ethoxycarbonylamino)benzene, 4-chloro-1,2-di(ethoxycarbonylamino)benzene, 2-chloro-1,3-di(ethoxycarbonylamino)benzene, 4-chloro-1,3-di(ethoxycarbonylamino)benzene, 5-chloro-1,3-di(ethoxycarbonylamino)benzene, 2-chloro-1,4-di(ethoxycarbonylamino)benzene, 3-chloro-1,4-di(ethoxycarbonylamino)benzene,
2-chloro-1-(t-butoxycarbonylamino)benzene, 3-chloro-1-(t-butoxycarbonylamino)benzene, 4-chloro-1-(t-butoxycarbonylamino)benzene, 3-chloro-1,2-di(t-butoxycarbonylamino)benzene, 4-chloro-1,2-di(t-butoxycarbonylamino)benzene, 2-chloro-1,3-di(t-butoxycarbonylamino)benzene, 4-chloro-1,3-di(t-butoxycarbonylamino)benzene, 5-chloro-1,3-di(t-butoxycarbonylamino)benzene, 2-chloro-1,4-di(t-butoxycarbonylamino)benzene, 3-chloro-1,4-di(t-butoxycarbonylamino)benzene,
2-chloro-1-(benzyloxycarbonylamino)benzene, 3-chloro-1-(benzyloxycarbonylamino)benzene, 4-chloro-1-(benzyloxycarbonylamino)benzene, 3-chloro-1,2-di(benzyloxycarbonylamino)benzene, 4-chloro-1,2-di(benzyloxycarbonylamino)benzene, 2-chloro-1,3-di(benzyloxycarbonylamino)benzene, 4-chloro-1,3-di(benzyloxycarbonylamino)benzene, 5-chloro-1,3-di(benzyloxycarbonylamino)benzene, 2-chloro-1,4-di(benzyloxycarbonylamino)benzene, 3-chloro-1,4-di(benzyloxycarbonylamino)benzene,
2-chloro-1-(p-methoxyphenoxycarbonylamino)benzene, 3-chloro-1-(p-methoxyphenoxycarbonylamino)benzene, 4-chloro-1-(p-methoxyphenoxycarbonylamino)benzene, 3-chloro-1,2-di(p-methoxyphenoxycarbonylamino)benzene, 4-chloro-1,2-di(p-methoxyphenoxycarbonylamino)benzene, 2-chloro-1,3-di(p-methoxyphenoxycarbonylamino)benzene, 4-chloro-1,3-di(p-methoxyphenoxycarbonylamino)benzene, 5-chloro-1,3-di(p-methoxyphenoxycarbonylamino)benzene, 2-chloro-1,4-di(p-methoxyphenoxycarbonylamino)benzene, 3-chloro-1,4-di(p-methoxyphenoxycarbonylamino)benzene,
2-chloro-1-(acetylamino)benzene, 3-chloro-1-(acetylamino)benzene, 4-chloro-1-(acetylamino)benzene, 3-chloro-1,2-di(acetylamino)benzene, 4-chloro-1,2-di(acetylamino)benzene, 2-chloro-1,3-di(acetylamino)benzene, 4-chloro-1,3-di(acetylamino)benzene, 5-chloro-1,3-di(acetylamino)benzene, 2-chloro-1,4-di(acetylamino)benzene, 3-chloro-1,4-di(acetylamino)benzene,
2-chloro-1-(trimethylacetylamino)benzene, 3-chloro-1-(trimethylacetylamino)benzene, 4-chloro-1-(trimethylacetylamino)benzene, 3-chloro-1,2-di(trimethylacetylamino)benzene, 4-chloro-1,2-di(trimethylacetylamino)benzene, 2-chloro-1,3-di(trimethylacetylamino)benzene, 4-chloro-1,3-di(trimethylacetylamino)benzene, 5-chloro-1,3-di(trimethylacetylamino)benzene, 2-chloro-1,4-di(trimethylacetylamino)benzene, 3-chloro-1,4-di(trimethylacetylamino)benzene,
2-chloro-1-(benzoylamino)benzene, 3-chloro-1-(benzoylamino)benzene, 4-chloro-1-(benzoylamino)benzene, 3-chloro-1,2-di(benzoylamino)benzene, 4-chloro-1,2-di(benzoylamino)benzene, 2-chloro-1,3-di(benzoylamino)benzene, 4-chloro-1,3-di(benzoylamino)benzene, 5-chloro-1,3-di(benzoylamino)benzene, 2-chloro-1,4-di(benzoylamino)benzene, 3-chloro-1,4-di(benzoylamino)benzene,
2-chloro-1-(p-methoxybenzoylamino)benzene, 3-chloro-1-(p-methoxybenzoylamino)benzene, 4-chloro-1-(p-methoxybenzoylamino)benzene, 3-chloro-1,2-di(p-methoxybenzoylamino)benzene, 4-chloro-1,2-di(p-methoxybenzoylamino)benzene, 2-chloro-1,3-di(p-methoxybenzoylamino)benzene, 4-chloro-1,3-di(p-methoxybenzoylamino)benzene, 5-chloro-1,3-di(p-methoxybenzoylamino)benzene, 2-chloro-1,4-di(p-methoxybenzoylamino)benzene, 3-chloro-1,4-di(p-methoxybenzoylamino)benzene,
2-chloro-1-(benzenesulfonylamino)benzene, 3-chloro-1-(benzenesulfonylamino)benzene, 4-chloro-1-(benzenesulfonylamino)benzene, 3-chloro-1,2-di(benzenesulfonylamino)benzene, 4-chloro-1,2-di(benzenesulfonylamino)benzene, 2-chloro-1,3-di(benzenesulfonylamino)benzene, 4-chloro-1,3-di(benzenesulfonylamino)benzene, 5-chloro-1,3-di(benzenesulfonylamino)benzene, 2-chloro-1,4-di(benzenesulfonylamino)benzene, 3-chloro-1,4-di(benzenesulfonylamino)benzene,
2-chloro-1-(methanesulfonylamino)benzene, 3-chloro-1-(methanesulfonylamino)benzene, 4-chloro-1-(methanesulfonylamino)benzene, 3-chloro-1,2-di(methanesulfonylamino)benzene, 4-chloro-1,2-di(methanesulfonylamino)benzene, 2-chloro-1,3-di(methanesulfonylamino)benzene, 4-chloro-1,3-di(methanesulfonylamino)benzene, 5-chloro-1,3-di(methanesulfonylamino)benzene, 2-chloro-1,4-di(methanesulfonylamino)benzene, 3-chloro-1,4-di(methanesulfonylamino)benzene,
2-chlorobenzylamine, 3-chlorobenzylamine, 4-chlorobenzylamine, 2-chlorophenethylamine, 3-chlorophenethylamine, 4-chlorophenethylamine, 3-(2-chlorophenyl)-1,-propylamine, 3-(3-chlorophenyl)-1,-propylamine, 3-(4-chlorophenyl)-1,-propylamine, 3-(2-chlorophenyl)-2-propylamine, 3-(3-chlorophenyl)-2-propylamine, 3-(4-chlorophenyl)-2-propylamine, 4-(2-chlorophenyl)-1,-butylamine, 4-(3-chlorophenyl)-1,-butylamine, 4-(4-chlorophenyl)-1,-butylamine,
2-chlorobenzaldehyde, 3-chlorobenzaldehyde, 4-chlorobenzaldehyde, 1-chloro-2,3-diformylbenzene, 1-chloro-2,4-diformylbenzene, 1-chloro-2,5-diformylbenzene, 1-chloro-2,6-diformylbenzene,
2-chloroacetophenone, 3-chloroacetophenone, 4-chloroacetophenone, 1-chloro-2,3-diacetylbenzene, 1-chloro-2,4-diacetylbenzene, 1-chloro-2,5-diacetylbenzene, 1-chloro-2,6-diacetylbenzene,
1-chloro-2-(trifluoroacetyl)benzene, 1-chloro-3-(trifluoroacetyl)benzene, 1-chloro-4-(trifluoroacetyl)benzene, 1-chloro-2,3-bis(trifluoroacetyl)benzene, 1-chloro-2,4-bis(trifluoroacetyl)benzene, 1-chloro-2,5-bis(trifluoroacetyl)benzene, 1-chloro-2,6-bis(trifluoroacetyl)benzene,
1-carbamoyl-2-chlorobenzene, 1-carbamoyl3-chlorobenzene, 1-carbamoyl-4-chlorobenzene, 1,2-dicarbamoyl3-chlorobenzene, 1,2-dicarbamoyl-4-chlorobenzene, 1,3-dicarbamoyl-2-chlorobenzene, 1,3-dicarbamoyl-4-chlorobenzene, 1,3-dicarbamoyl5-chlorobenzene, 1,4-dicarbamoyl-2-chlorobenzene, 1,4-dicarbamoyl3-chlorobenzene,
1-(N-methylcarbamoyl)-2-chlorobenzene, 1-(N-methylcarbamoyl)-3-chlorobenzene, 1-(N-methylcarbamoyl)-4-chlorobenzene, 1,2-di(N-methylcarbamoyl)-3-chlorobenzene, 1,2-di(N-methylcarbamoyl)-4-chlorobenzene, 1,3-di(N-methylcarbamoyl)-2-chlorobenzene, 1,3-di(N-methylcarbamoyl)-4-chlorobenzene, 1,3-di(N-methylcarbamoyl)-5-chlorobenzene, 1,4-di(N-methylcarbamoyl)-2-chlorobenzene, 1,4-di(N-methylcarbamoyl)-3-chlorobenzene,
1-(N-phenylcarbamoyl)-2-chlorobenzene, 1-(N-phenylcarbamoyl)-3-chlorobenzene, 1-(N-phenylcarbamoyl)-4-chlorobenzene, 1,2-di(N-phenylcarbamoyl)-3-chlorobenzene, 1,2-di(N-phenylcarbamoyl)-4-chlorobenzene, 1,3-di(N-phenylcarbamoyl)-2-chlorobenzene, 1,3-di(N-phenylcarbamoyl)-4-chlorobenzene, 1,3-di(N-phenylcarbamoyl)-5-chlorobenzene, 1,4-di(N-phenylcarbamoyl)-2-chlorobenzene, 1,4-di(N-phenylcarbamoyl)-3-chlorobenzene,
1-(N,N-dimethylcarbamoyl)-2-chlorobenzene, 1-(N,N-dimethylcarbamoyl)-3-chlorobenzene, 1-(N,N-dimethylcarbamoyl)-4-chlorobenzene, 1,2-bis(N,N-dimethylcarbamoyl)-3-chlorobenzene, 1,2-bis(N,N-dimethylcarbamoyl)-4-chlorobenzene, 1,3-bis(N,N-dimethylcarbamoyl)-2-chlorobenzene, 1,3-bis(N,N-dimethylcarbamoyl)-4-chlorobenzene, 1,3-bis(N,N-dimethylcarbamoyl)-5-chlorobenzene, 1,4-bis(N,N-dimethylcarbamoyl)-2-chlorobenzene, 1,4-bis(N,N-dimethylcarbamoyl)-3-chlorobenzene,
1-(2-chlorophenyl)pyrrolidine-2,5-dione, 1-(3-chlorophenyl)pyrrolidine-2,5-dione, 1-(4-chlorophenyl)pyrrolidine-2,5-dione,
2-(2-chlorophenyl)isoindoline-1,3-dione, 2-(3-chlorophenyl)isoindoline-1,3-dione, 2-(4-chlorophenyl)isoindoline-1,3-dione,
2-chlorothiophene, 3-chlorothiophene, 2-chloro-3-formylthiophene, 3-chloro-2-formylthiophene, 2-chloro-3-methylthiophene, 3-chloro-2-methylthiophene, 3-acetyl-2-chlorothiophene, 2-acetyl-3-chlorothiophene,
2-chlorofuran, 3-chlorofuran, 2-chloropyrrole, 3-chloropyrrole, 2-chloroimidazole, 4-chloroimidazole, 2-chloropyridine, 3-chloropyridine, 4-chloropyridine,
1-chloronaphthalene, 2-chloronaphthalene, 1-chloroanthracene, 2-chloroanthracene, 3-chloroanthracene, 4-chloroanthracene, 1-chlorophenanthrene, 2-chlorophenanthrene, 3-chlorophenanthrene, 4-chlorophenanthrene,
1-chlorofluorene, 2-chlorofluorene, 3-chlorofluorene, 4-chlorofluorene, 4-chloroindene, 5-chloroindene, 6-chloroindene, 7-chloroindene,
2-chloroquinoline, 3-chloroquinoline, 4-chloroquinoline, 5-chloroquinoline, 6-chloroquinoline, 7-chloroquinoline, 8-chloroquinoline, 1-chloroisoquinoline, 3-chloroisoquinoline, 4-chloroisoquinoline, 5-chloroisoquinoline, 6-chloroisoquinoline, 7-chloroisoquinoline, 8-chloroisoquinoline,
4-chlorobenzo[d][1,3]dioxol, 5-chlorobenzo[d][1,3]dioxol, 5-chloro-2,3-dihydrobenzo[b][1,4]dioxin, 6-chloro-2,3-dihydrobenzo[b][1,4]dioxin,
2-chlorobiphenyl, 3-chlorobiphenyl, 4-chlorobiphenyl, 4'-methyl-2-chlorobiphenyl, 4'-methyl-3-chlorobiphenyl, 4'-methyl-4-chlorobiphenyl, 4'-ethyl-2-chlorobiphenyl, 4'-ethyl-3-chlorobiphenyl, 4'-ethyl-4-chlorobiphenyl, and compounds obtained by substitution of "chloro group" in the above-described compounds by "bromo group", "iodo group", "mesyl group", "trifluoromethanesulfonyl group", "tosyl group", and the like. In the case of compounds having an amino group or alkylamino group, these compounds may form salts, for example, together with a mineral acid such as hydrochloric acid, sulfuric acid and the like.

As these aromatic organic compounds (1), commercially available compounds may be used or those produced by any known methods may be used.

Specific examples of the organoboron compound (2) and the boroxine compound (3) include the following compounds.
phenylboronic acid, 2-methylphenylboronic acid, 3-methylphenylboronic acid, 4-methylphenylboronic acid, 2,3-dimethylphenylboronic acid, 2,4-dimethylphenylboronic acid, 2,5-dimethylphenylboronic acid, 2,6-dimethylphenylboronic acid, 3,5-dimethylphenylboronic acid, 3,4-dimethylphenylboronic acid, 2-ethylboronic acid, 3-ethylboronic acid, 4-ethylphenylboronic acid, 2,3-diethylphenylboronic acid, 2,4-diethylphenylboronic acid, 2,5-diethylphenylboronic acid, 2,6-diethylphenylboronic acid, 3,5-diethylphenylboronic acid, 3,4-diethylphenylboronic acid, 2-ethyl-3-methylphenylboronic acid, 3-ethyl-2-methylphenylboronic acid, 2-ethyl-6-methylphenylboronic acid, 3-ethyl-5-methylphenylboronic acid, 4-n-propylphenylboronic acid, 4-isopropylphenylboronic acid, 2-n-propylphenylboronic acid, 2-isopropylphenylboronic acid, 3-n-propylphenylboronic acid, 3-isopropylphenylboronic acid, 4-n-butylphenylboronic acid, 2-(1-methylpropyl)phenylboronic acid, 3-(1-methylpropyl)phenylboronic acid, 4-(1-methylpropyl)phenylboronic acid, 1-(2-methylpropyl)phenylboronic acid, 2-(2-methylpropyl)phenylboronic acid, 3-(2-methylpropyl)phenylboronic acid, 4-(2-methylpropyl)phenylboronic acid, 4-t-butylphenylboronic acid,
2-aminophenylboronic acid, 3-aminophenylboronic acid, 4-aminophenylboronic acid, 2-amino-3-methylphenylboronic acid, 2-amino-4-methylphenylboronic acid, 2-amino-5-methylphenylboronic acid, 2-amino-6-methylphenylboronic acid, 3-amino-2-methylphenylboronic acid, 3-amino-4-methylphenylboronic acid, 3-amino-5-methylphenylboronic acid, 4-amino-6-methylphenylboronic acid, 4-amino-5-methylphenylboronic acid,
2-amino-3-ethylphenylboronic acid, 2-amino-4-ethylphenylboronic acid, 2-amino-5-ethylphenylboronic acid, 3-amino-2-ethylphenylboronic acid, 3-amino-4-ethylphenylboronic acid, 3-amino-5-ethylphenylboronic acid, 4-amino-6-ethylphenylboronic acid, 4-amino-5-ethylphenylboronic acid,
2-amino-3-n-propylphenylboronic acid, 2-amino-4-n-propylphenylboronic acid, 2-amino-5-n-propylphenylboronic acid, 2-amino-6-n-propylphenylboronic acid, 3-amino-4-n-propylphenylboronic acid, 3-amino-5-n-propylphenylboronic acid,
3-amino-6-n-propylphenylboronic acid, 4-amino-6-n-propylphenylboronic acid, 4-amino-5-n-propylphenylboronic acid, 2-amino-3-isopropylphenylboronic acid, 2-amino-4-isopropylphenylboronic acid, 2-amino-5-isopropylphenylboronic acid, 2-amino-6-isopropylphenylboronic acid, 3-amino-4-isopropylphenylboronic acid, 3-amino-5-isopropylphenylboronic acid, 3-amino-6-isopropylphenylboronic acid, 4-amino-6-isopropylphenylboronic acid, 4-amino-5-isopropylphenylboronic acid, 2-amino-3-n-butylphenylboronic acid, 2-amino-4-n-butylphenylboronic acid, 2-amino-5-n-butylphenylboronic acid, 2-amino-6-n-butylphenylboronic acid, 3-amino-4-n-butylphenylboronic acid, 3-amino-5-n-butylphenylboronic acid, 3-amino-6-n-butylphenylboronic acid, 4-amino-6-n-butylphenylboronic acid, 4-amino-5-n-butylphenylboronic acid,
2-amino-3-t-butylphenylboronic acid, 2-amino-4-t-butylphenylboronic acid, 2-amino-5-t-butylphenylboronic acid, 2-amino-6-t-butylphenylboronic acid, 3-amino-4-t-butylphenylboronic acid, 3-amino-5-t-butylphenylboronic acid, 3-amino-6-t-butylphenylboronic acid, 4-amino-6-t-butylphenylboronic acid, 4-amino-5-t-butylphenylboronic acid,
2-fluorophenylboronic acid, 3-fluorophenylboronic acid, 4-fluorophenylboronic acid, 2,3-difluorophenylboronic acid, 2,4-difluorophenylboronic acid, 2,5-difluorophenylboronic acid, 2,6-difluorophenylboronic acid, 3,5-difluorophenylboronic acid, 3,4-difluorophenylboronic acid,
2-amino-3-fluorophenylboronic acid, 2-amino-4-fluorophenylboronic acid, 2-amino-5-fluorophenylboronic acid, 3-amino-2-fluorophenylboronic acid, 3-amino-4-fluorophenylboronic acid, 3-amino-5-fluorophenylboronic acid, 4-amino-6-fluorophenylboronic acid, 4-amino-5-fluorophenylboronic acid,
2-trifluoromethylphenylboronic acid, 3-trifluoromethylphenylboronic acid, 4-trifluoromethylphenylboronic acid, 2,3-ditrifluoromethylphenylboronic acid, 2,4-ditrifluoromethylphenylboronic acid, 2,5-ditrifluoromethylphenylboronic acid, 2,6-ditrifluoromethylphenylboronic acid, 3,5-ditrifluoromethylphenylboronic acid, 3,4-ditrifluoromethylphenylboronic acid,
2-amino-3-trifluoromethylphenylboronic acid, 2-amino-4-trifluoromethylphenylboronic acid, 2-amino-5-trifluoromethylphenylboronic acid, 3-amino-2-trifluoromethylphenylboronic acid, 3-amino-4-trifluoromethylphenylboronic acid, 3-amino-5-trifluoromethylphenylboronic acid, 4-amino-6-trifluoromethylphenylboronic acid, 4-amino-5-trifluoromethylphenylboronic acid,
2-cyclopropylphenylboronic acid, 3-cyclopropylphenylboronic acid, 4-cyclopropylphenylboronic acid, 2,3-dicyclopropylphenylboronic acid, 2,4-dicyclopropylphenylboronic acid, 2,5-dicyclopropylphenylboronic acid, 2,6-dicyclopropylphenylboronic acid, 3,5-dicyclopropylphenylboronic acid, 3,4-dicyclopropylphenylboronic acid,
2-amino-3-cyclopropylphenylboronic acid, 2-amino-4-cyclopropylphenylboronic acid, 2-amino-5-cyclopropylphenylboronic acid, 3-amino-2-cyclopropylphenylboronic acid, 3-amino-4-cyclopropylphenylboronic acid, 3-amino-5-cyclopropylphenylboronic acid, 4-amino-6-cyclopropylphenylboronic acid, 4-amino-5-cyclopropylphenylboronic acid,
2-cyclobutylphenylboronic acid, 3-cyclobutylphenylboronic acid, 4-cyclobutylphenylboronic acid, 2,3-dicyclobutylphenylboronic acid, 2,4-dicyclobutylphenylboronic acid, 2,5-dicyclobutylphenylboronic acid, 2,6-dicyclobutylphenylboronic acid, 3,5-dicyclobutylphenylboronic acid, 3,4-dicyclobutylphenylboronic acid,
2-amino-3-cyclobutylphenylboronic acid, 2-amino-4-cyclobutylphenylboronic acid, 2-amino-5-cyclobutylphenylboronic acid, 3-amino-2-cyclobutylphenylboronic acid, 3-amino-4-cyclobutylphenylboronic acid, 3-amino-5-cyclobutylphenylboronic acid, 4-amino-6-cyclobutylphenylboronic acid, 4-amino-5-cyclobutylphenylboronic acid,
2-cyclopentylphenylboronic acid, 3-cyclopentylphenylboronic acid, 4-cyclopentylphenylboronic acid, 2,3-dicyclopentylphenylboronic acid, 2,4-dicyclopentylphenylboronic acid, 2,5-dicyclopentylphenylboronic acid, 2,6-dicyclopentylphenylboronic acid, 3,5-dicyclopentylphenylboronic acid, 3,4-dicyclopentylphenylboronic acid,
2-amino-3-cyclopentylphenylboronic acid, 2-amino-4-cyclopentylphenylboronic acid, 2-amino-5-cyclopentylphenylboronic acid, 3-amino-2-cyclopentylphenylboronic acid, 3-amino-4-cyclopentylphenylboronic acid, 3-amino-5-cyclopentylphenylboronic acid, 4-amino-6-cyclopentylphenylboronic acid, 4-amino-5-cyclopentylphenylboronic acid,
2-cyclohexylphenylboronic acid, 3-cyclohexylphenylboronic acid, 4-cyclohexylphenylboronic acid, 2,3-dicyclohexylphenylboronic acid, 2,4-dicyclohexylphenylboronic acid, 2,5-dicyclohexylphenylboronic acid, 2,6-dicyclohexylphenylboronic acid, 3,5-dicyclohexylphenylboronic acid, 3,4-dicyclohexylphenylboronic acid,
2-amino-3-cyclohexylphenylboronic acid, 2-amino-4-cyclohexylphenylboronic acid, 2-amino-5-cyclohexylphenylboronic acid, 3-amino-2-cyclohexylphenylboronic acid, 3-amino-4-cyclohexylphenylboronic acid, 3-amino-5-cyclohexylphenylboronic acid, 4-amino-6-cyclohexylphenylboronic acid, 4-amino-5-cyclohexylphenylboronic acid,
2-hydroxyphenylboronic acid, 3-hydroxyphenylboronic acid, 4-hydroxyphenylboronic acid, 2,3-dihydroxyphenylboronic acid, 2,4-dihydroxyphenylboronic acid, 2,5-dihydroxyphenylboronic acid, 2,6-dihydroxyphenylboronic acid, 3,5-dihydroxyphenylboronic acid, 3,4-dihydroxyphenylboronic acid,
2-amino-3-hydroxyphenylboronic acid, 2-amino-4-hydroxyphenylboronic acid, 2-amino-5-hydroxyphenylboronic acid, 3-amino-2-hydroxyphenylboronic acid, 3-amino-4-hydroxyphenylboronic acid, 3-amino-5-hydroxyphenylboronic acid, 4-amino-6-hydroxyphenylboronic acid, 4-amino-5-hydroxyphenylboronic acid,
2-hydroxymethylphenylboronic acid, 3-hydroxymethylphenylboronic acid, 4-hydroxymethylphenylboronic acid, 2,3-dihydroxymethylphenylboronic acid, 2,4-dihydroxymethylphenylboronic acid, 2,5-dihydroxymethylphenylboronic acid, 2,6-dihydroxymethylphenylboronic acid, 3,5-dihydroxymethylphenylboronic acid, 3,4-dihydroxymethylphenylboronic acid,
2-amino-3-hydroxymethylphenylboronic acid, 2-amino-4-hydroxymethylphenylboronic acid, 2-amino-5-hydroxymethylphenylboronic acid, 3-amino-2-hydroxymethylphenylboronic acid, 3-amino-4-hydroxymethylphenylboronic acid, 3-amino-5-hydroxymethylphenylboronic acid, 4-amino-6-hydroxymethylphenylboronic acid, 4-amino-5-hydroxymethylphenylboronic acid,
2-methoxyphenylboronic acid, 3-methoxyphenylboronic acid, 4-methoxyphenylboronic acid, 2,3-dimethoxyphenylboronic acid, 2,4-dimethoxyphenylboronic acid, 2,5-dimethoxyphenylboronic acid, 2,6-dimethoxyphenylboronic acid, 3,5-dimethoxyphenylboronic acid, 3,4-dimethoxyphenylboronic acid,
2-amino-3-methoxyphenylboronic acid, 2-amino-4-methoxyphenylboronic acid, 2-amino-5-methoxyphenylboronic acid, 3-amino-2-methoxyphenylboronic acid, 3-amino-4-methoxyphenylboronic acid, 3-amino-5-methoxyphenylboronic acid, 4-amino-6-methoxyphenylboronic acid, 4-amino-5-methoxyphenylboronic acid,
2-ethoxyphenylboronic acid, 3-ethoxyphenylboronic acid, 4-ethoxyphenylboronic acid, 2,3-diethoxyphenylboronic acid, 2,4-diethoxyphenylboronic acid, 2,5-diethoxyphenylboronic acid, 2,6-diethoxyphenylboronic acid, 3,5-diethoxyphenylboronic acid, 3,4-diethoxyphenylboronic acid,
2-amino-3-ethoxyphenylboronic acid, 2-amino-4-ethoxyphenylboronic acid, 2-amino-5-ethoxyphenylboronic acid, 3-amino-2-ethoxyphenylboronic acid, 3-amino-4-ethoxyphenylboronic acid, 3-amino-5-ethoxyphenylboronic acid, 4-amino-6-ethoxyphenylboronic acid, 4-amino-5-ethoxyphenylboronic acid,
2-propoxyphenylboronic acid, 3-propoxyphenylboronic acid, 4-propoxyphenylboronic acid, 2,3-dipropoxyphenylboronic acid, 2,4-dipropoxyphenylboronic acid, 2,5-dipropoxyphenylboronic acid, 2,6-dipropoxyphenylboronic acid, 3,5-dipropoxyphenylboronic acid, 3,4-dipropoxyphenylboronic acid,
2-amino-3-propoxyphenylboronic acid, 2-amino-4-propoxyphenylboronic acid, 2-amino-5-propoxyphenylboronic acid, 3-amino-2-propoxyphenylboronic acid, 3-amino-4-propoxyphenylboronic acid, 3-amino-5-propoxyphenylboronic acid, 4-amino-6-propoxyphenylboronic acid, 4-amino-5-propoxyphenylboronic acid,
2-butoxyphenylboronic acid, 3-butoxyphenylboronic acid, 4-butoxyphenylboronic acid, 2,3-dibutoxyphenylboronic acid, 2,4-dibutoxyphenylboronic acid, 2,5-dibutoxyphenylboronic acid, 2,6-dibutoxyphenylboronic acid, 3,5-dibutoxyphenylboronic acid, 3,4-dibutoxyphenylboronic acid,
2-amino-3-butoxyphenylboronic acid, 2-amino-4-butoxyphenylboronic acid, 2-amino-5-butoxyphenylboronic acid, 3-amino-2-butoxyphenylboronic acid, 3-amino-4-butoxyphenylboronic acid, 3-amino-5-butoxyphenylboronic acid, 4-amino-6-butoxyphenylboronic acid, 4-amino-5-butoxyphenylboronic acid,
2-t-butoxyphenylboronic acid, 3-t-butoxyphenylboronic acid, 4-t-butoxyphenylboronic acid, 2,3-di-t-butoxyphenylboronic acid, 2,4-di-t-butoxyphenylboronic acid, 2,5-di-t-butoxyphenylboronic acid, 2,6-di-t-butoxyphenylboronic acid, 3,5-di-t-butoxyphenylboronic acid, 3,4-di-t-butoxyphenylboronic acid,
2-amino-3-t-butoxyphenylboronic acid, 2-amino-4-t-butoxyphenylboronic acid, 2-amino-5-t-butoxyphenylboronic acid, 3-amino-2-t-butoxyphenylboronic acid, 3-amino-4-t-butoxyphenylboronic acid, 3-amino-5-t-butoxyphenylboronic acid, 4-amino-6-t-butoxyphenylboronic acid, 4-amino-5-t-butoxyphenylboronic acid,
2-phenoxyphenylboronic acid, 3-phenoxyphenylboronic acid, 4-phenoxyphenylboronic acid, 2,3-diphenoxyphenylboronic acid, 2,4-diphenoxyphenylboronic acid, 2,5-diphenoxyphenylboronic acid, 2,6-diphenoxyphenylboronic acid, 3,5-diphenoxyphenylboronic acid, 3,4-diphenoxyphenylboronic acid,
2-amino-3-phenoxyphenylboronic acid, 2-amino-4-phenoxyphenylboronic acid, 2-amino-5-phenoxyphenylboronic acid, 3-amino-2-phenoxyphenylboronic acid, 3-amino-4-phenoxyphenylboronic acid, 3-amino-5-phenoxyphenylboronic acid, 4-amino-6-phenoxyphenylboronic acid, 4-amino-5-phenoxyphenylboronic acid,
2-mercaptophenylboronic acid, 3-mercaptophenylboronic acid, 4-mercaptophenylboronic acid, 2,3-dimercaptophenylboronic acid, 2,4-dimercaptophenylboronic acid, 2,5-dimercaptophenylboronic acid, 2,6-dimercaptophenylboronic acid, 3,5-dimercaptophenylboronic acid, 3,4-dimercaptophenylboronic acid,
2-amino-3-mercaptophenylboronic acid, 2-amino-4-mercaptophenylboronic acid, 2-amino-5-mercaptophenylboronic acid, 3-amino-2-mercaptophenylboronic acid, 3-amino-4-mercaptophenylboronic acid, 3-amino-5-mercaptophenylboronic acid, 4-amino-6-mercaptophenylboronic acid, 4-amino-5-mercaptophenylboronic acid,
2-methylthiophenylboronic acid, 3-methylthiophenylboronic acid, 4-methylthiophenylboronic acid, 2,3-dimethylthiophenylboronic acid, 2,4-dimethylthiophenylboronic acid, 2,5-dimethylthiophenylboronic acid, 2,6-dimethylthiophenylboronic acid, 3,5-dimethylthiophenylboronic acid, 3,4-dimethylthiophenylboronic acid,
2-amino-3-methylthiophenylboronic acid, 2-amino-4-methylthiophenylboronic acid, 2-amino-5-methylthiophenylboronic acid, 3-amino-2-methylthiophenylboronic acid, 3-amino-4-methylthiophenylboronic acid, 3-amino-5-methylthiophenylboronic acid, 4-amino-6-methylthiophenylboronic acid, 4-amino-5-methylthiophenylboronic acid,
2-ethylthiophenylboronic acid, 3-ethylthiophenylboronic acid, 4-ethylthiophenylboronic acid, 2,3-diethylthiophenylboronic acid, 2,4-diethylthiophenylboronic acid, 2,5-diethylthiophenylboronic acid, 2,6-diethylthiophenylboronic acid, 3,5-diethylthiophenylboronic acid, 3,4-diethylthiophenylboronic acid,
2-amino-3-ethylthiophenylboronic acid, 2-amino-4-ethylthiophenylboronic acid, 2-amino-5-ethylthiophenylboronic acid, 3-amino-2-ethylthiophenylboronic acid, 3-amino-4-ethylthiophenylboronic acid, 3-amino-5-ethylthiophenylboronic acid, 4-amino-6-ethylthiophenylboronic acid, 4-amino-5-ethylthiophenylboronic acid,
2-phenylthiophenylboronic acid, 3-phenylthiophenylboronic acid, 4-phenylthiophenylboronic acid, 2,3-diphenylthiophenylboronic acid, 2,4-diphenylthiophenylboronic acid, 2,5-diphenylthiophenylboronic acid, 2,6-diphenylthiophenylboronic acid, 3,5-diphenylthiophenylboronic acid, 3,4-diphenylthiophenylboronic acid,
2-amino-3-phenylthiophenylboronic acid, 2-amino-4-phenylthiophenylboronic acid, 2-amino-5-phenylthiophenylboronic acid, 3-amino-2-phenylthiophenylboronic acid, 3-amino-4-phenylthiophenylboronic acid, 3-amino-5-phenylthiophenylboronic acid, 4-amino-6-phenylthiophenylboronic acid, 4-amino-5-phenylthiophenylboronic acid,
2-methylaminophenylboronic acid, 3-methylaminophenylboronic acid, 4-methylaminophenylboronic acid, 2,3-dimethylaminophenylboronic acid, 2,4-dimethylaminophenylboronic acid, 2,5-dimethylaminophenylboronic acid, 2,6-dimethylaminophenylboronic acid, 3,5-dimethylaminophenylboronic acid, 3,4-dimethylaminophenylboronic acid,
2-amino-3-methylaminophenylboronic acid, 2-amino-4-methylaminophenylboronic acid, 2-amino-5-methylaminophenylboronic acid, 3-amino-2-methylaminophenylboronic acid, 3-amino-4-methylaminophenylboronic acid, 3-amino-5-methylaminophenylboronic acid, 4-amino-6-methylaminophenylboronic acid, 4-amino-5-methylaminophenylboronic acid,
2-ethylaminophenylboronic acid, 3-ethylaminophenylboronic acid, 4-ethylaminophenylboronic acid, 2,3-diethylaminophenylboronic acid, 2,4-diethylaminophenylboronic acid, 2,5-diethylaminophenylboronic acid, 2,6-diethylaminophenylboronic acid, 3,5-diethylaminophenylboronic acid, 3,4-diethylaminophenylboronic acid,
2-amino-3-ethylaminophenylboronic acid, 2-amino-4-ethylaminophenylboronic acid, 2-amino-5-ethylaminophenylboronic acid, 3-amino-2-ethylaminophenylboronic acid, 3-amino-4-ethylaminophenylboronic acid, 3-amino-5-ethylaminophenylboronic acid, 4-amino-6-ethylaminophenylboronic acid, 4-amino-5-ethylaminophenylboronic acid,
2-dimethylaminophenylboronic acid, 3-dimethylaminophenylboronic acid, 4-dimethylaminophenylboronic acid, 2,3-dimethylaminophenylboronic acid, 2,4-dimethylaminophenylboronic acid, 2,5-dimethylaminophenylboronic acid, 2,6-dimethylaminophenylboronic acid, 3,5-dimethylaminophenylboronic acid, 3,4-dimethylaminophenylboronic acid,
2-amino-3-dimethylaminophenylboronic acid, 2-amino-4-dimethylaminophenylboronic acid, 2-amino-5-dimethylaminophenylboronic acid, 3-amino-2-dimethylaminophenylboronic acid, 3-amino-4-dimethylaminophenylboronic acid, 3-amino-5-dimethylaminophenylboronic acid, 4-amino-6-dimethylaminophenylboronic acid, 4-amino-5-dimethylaminophenylboronic acid,
2-cyclopropylaminophenylboronic acid, 3-cyclopropylaminophenylboronic acid, 4-cyclopropylaminophenylboronic acid, 2,3-dicyclopropylaminophenylboronic acid, 2,4-dicyclopropylaminophenylboronic acid, 2,5-dicyclopropylaminophenylboronic acid, 2,6-dicyclopropylaminophenylboronic acid, 3,5-dicyclopropylaminophenylboronic acid, 3,4-dicyclopropylaminophenylboronic acid,
2-cyclohexylaminophenylboronic acid, 3-cyclohexylaminophenylboronic acid, 4-cyclohexylaminophenylboronic acid, 2,3-dicyclohexylaminophenylboronic acid, 2,4-dicyclohexylaminophenylboronic acid, 2,5-dicyclohexylaminophenylboronic acid, 2,6-dicyclohexylaminophenylboronic acid, 3,5-dicyclohexylaminophenylboronic acid, 3,4-dicyclohexylaminophenylboronic acid,
2-amino-3-cyclohexylaminophenylboronic acid, 2-amino-4-cyclohexylaminophenylboronic acid, 2-amino-5-cyclohexylaminophenylboronic acid, 3-amino-2-cyclohexylaminophenylboronic acid, 3-amino-4-cyclohexylaminophenylboronic acid, 3-amino-5-cyclohexylaminophenylboronic acid, 4-amino-6-cyclohexylaminophenylboronic acid, 4-amino-5-cyclohexylaminophenylboronic acid,
2-methoxycarbonylaminophenylboronic acid, 3-methoxycarbonylaminophenylboronic acid, 4-methoxycarbonylaminophenylboronic acid, 2,3-dimethoxycarbonylaminophenylboronic acid, 2,4-dimethoxycarbonylaminophenylboronic acid, 2,5-dimethoxycarbonylaminophenylboronic acid, 2,6-dimethoxycarbonylaminophenylboronic acid, 3,5-dimethoxycarbonylaminophenylboronic acid, 3,4-dimethoxycarbonylaminophenylboronic acid,
2-ethoxycarbonylaminophenylboronic acid, 3-ethoxycarbonylaminophenylboronic acid, 4-ethoxycarbonylaminophenylboronic acid, 2,3-diethoxycarbonylaminophenylboronic acid, 2,4-diethoxycarbonylaminophenylboronic acid, 2,5-diethoxycarbonylaminophenylboronic acid, 2,6-diethoxycarbonylaminophenylboronic acid, 3,5-diethoxycarbonylaminophenylboronic acid, 3,4-diethoxycarbonylaminophenylboronic acid,
2-t-butoxycarbonylaminophenylboronic acid, 3-t-butoxycarbonylaminophenylboronic acid, 4-t-butoxycarbonylaminophenylboronic acid, 2,3-di-t-butoxycarbonylaminophenylboronic acid, 2,4-di-t-butoxycarbonylaminophenylboronic acid, 2,5-di-t-butoxycarbonylaminophenylboronic acid, 2,6-di-t-butoxycarbonylaminophenylboronic acid, 3,5-di-t-butoxycarbonylaminophenylboronic acid, 3,4-di-t-butoxycarbonylaminophenylboronic acid,
2-benzyloxycarbonylaminophenylboronic acid, 3-benzyloxycarbonylaminophenylboronic acid, 4-benzyloxycarbonylaminophenylboronic acid, 2,3-dibenzyloxycarbonylaminophenylboronic acid, 2,4-dibenzyloxycarbonylaminophenylboronic acid, 2,5-dibenzyloxycarbonylaminophenylboronic acid, 2,6-dibenzyloxycarbonylaminophenylboronic acid, 3,5-dibenzyloxycarbonylaminophenylboronic acid, 3,4-dibenzyloxycarbonylaminophenylboronic acid, 2-p-methoxyphenoxycarbonylaminophenylboronic acid, 3-p-methoxyphenoxycarbonylaminophenylboronic acid, 4-p-methoxyphenoxycarbonylaminophenylboronic acid, 2,3-dip-methoxyphenoxycarbonylaminophenylboronic acid, 2,4-di-p-methoxyphenoxycarbonylaminophenylboronic acid, 2,5-di-p-methoxyphenoxycarbonylaminophenylboronic acid, 2,6-di-p-methoxyphenoxycarbonylaminophenylboronic acid, 3,5-di-p-methoxyphenoxycarbonylaminophenylboronic acid, 3,4-di-p-methoxyphenoxycarbonylaminophenylboronic acid,
2-acetaminophenylboronic acid, 3-acetaminophenylboronic acid, 4-acetaminophenylboronic acid, 2,3-diacetaminophenylboronic acid, 2,4-diacetaminophenylboronic acid, 2,5-diacetaminophenylboronic acid, 2,6-diacetaminophenylboronic acid, 3,5-diacetaminophenylboronic acid, 3,4-diacetaminophenylboronic acid,
2-pivaloylaminophenylboronic acid, 3-pivaloylaminophenylboronic acid, 4-pivaloylaminophenylboronic acid, 2,3-dipivaloylaminophenylboronic acid, 2,4-dipivaloylaminophenylboronic acid, 2,5-dipivaloylaminophenylboronic acid, 2,6-dipivaloylaminophenylboronic acid, 3,5-dipivaloylaminophenylboronic acid, 3,4-dipivaloylaminophenylboronic acid,
2-benzoylaminophenylboronic acid, 3-benzoylaminophenylboronic acid, 4-benzoylaminophenylboronic acid, 2,3-dibenzoylaminophenylboronic acid, 2,4-dibenzoylaminophenylboronic acid, 2,5-dibenzoylaminophenylboronic acid, 2,6-dibenzoylaminophenylboronic acid, 3,5-dibenzoylaminophenylboronic acid, 3,4-dibenzoylaminophenylboronic acid,
2-p-methoxybenzoylaminophenylboronic acid, 3-p-methoxybenzoylaminophenylboronic acid, 4-p-methoxybenzoylaminophenylboronic acid, 2,3-di-p-methoxybenzoylaminophenylboronic acid, 2,4-di-p-methoxybenzoylaminophenylboronic acid, 2,5-di-p-methoxybenzoylaminophenylboronic acid, 2,6-di-p-methoxybenzoylaminophenylboronic acid, 3,5-di-p-methoxybenzoylaminophenylboronic acid, 3,4-di-p-methoxybenzoylaminophenylboronic acid,
2-benzenesulfoneamidephenylboronic acid, 3-benzenesulfoneamidephenylboronic acid, 4-benzenesulfoneamidephenylboronic acid, 2,3-dibenzenesulfoneamidephenylboronic acid, 2,4-dibenzenesulfoneamidephenylboronic acid, 2,5-dibenzenesulfoneamidephenylboronic acid, 2,6-dibenzenesulfoneamidephenylboronic acid, 3,5-dibenzenesulfoneamidephenylboronic acid, 3,4-dibenzenesulfoneamidephenylboronic acid,
2-methanesulfoneamidephenylboronic acid, 3-methanesulfoneamidephenylboronic acid, 4-methanesulfoneamidephenylboronic acid, 2,3-dmethanesulfoneamidephenylboronic acid, 2,4-dmethanesulfoneamidephenylboronic acid, 2,5-dmethanesulfoneamidephenylboronic acid, 2,6-dmethanesulfoneamidephenylboronic acid, 3,5-dmethanesulfoneamidephenylboronic acid, 3,4-dmethanesulfoneamidephenylboronic acid,
2-iminophenylboronic acid, 3-iminophenylboronic acid, 4-iminophenylboronic acid, 2,3-diiminophenylboronic acid, 2,4-diiminophenylboronic acid, 2,5-diiminophenylboronic acid, 2,6-diiminophenylboronic acid, 3,5-diiminophenylboronic acid, 3,4-diiminophenylboronic acid,
2-formylphenylboronic acid, 3-formylphenylboronic acid, 4-formylphenylboronic acid, 2,3-diformylphenylboronic acid, 2,4-diformylphenylboronic acid, 2,5-diformylphenylboronic acid, 2,6-diformylphenylboronic acid, 3,5-diformylphenylboronic acid, 3,4-diformylphenylboronic acid,
2-acetylphenylboronic acid, 3-acetylphenylboronic acid, 4-acetylphenylboronic acid, 2,3-diacetylphenylboronic acid, 2,4-diacetylphenylboronic acid, 2,5-diacetylphenylboronic acid, 2,6-diacetylphenylboronic acid, 3,5-diacetylphenylboronic acid, 3,4-diacetylphenylboronic acid,
2-trifluoroacetylphenylboronic acid, 3-trifluoroacetylphenylboronic acid, 4-trifluoroacetylphenylboronic acid, 2,3-ditrifluoroacetylphenylboronic acid, 2,4-ditrifluoroacetylphenylboronic acid, 2,5-ditrifluoroacetylphenylboronic acid, 2,6-ditrifluoroacetylphenylboronic acid, 3,5-ditrifluoroacetylphenylboronic acid, 3,4-ditrifluoroacetylphenylboronic acid,
2-acetamidephenylboronic acid, 3-acetamidephenylboronic acid, 4-acetamidephenylboronic acid, 2,3-diacetamidephenylboronic acid, 2,4-diacetamidephenylboronic acid, 2,5-diacetamidephenylboronic acid, 2,6-diacetamidephenylboronic acid, 3,5-diacetamidephenylboronic acid, 3,4-diacetamidephenylboronic acid,
2-carbamoylphenylboronic acid, 3-carbamoylphenylboronic acid, 4-carbamoylphenylboronic acid, 2,3-dicarbamoylphenylboronic acid, 2,4-dicarbamoylphenylboronic acid, 2,5-dicarbamoylphenylboronic acid, 2,6-dicarbamoylphenylboronic acid, 3,5-dicarbamoylphenylboronic acid, 3,4-dicarbamoylphenylboronic acid,
2-(N-phenylcarbamoyl)phenylboronic acid, 3-(N-phenylcarbamoyl)phenylboronic acid, 4-(N-phenylcarbamoyl)phenylboronic acid, 2,3-di(N-phenylcarbamoyl)phenylboronic acid, 2,4-di(N-phenylcarbamoyl)phenylboronic acid, 2,5-di(N-phenylcarbamoyl)phenylboronic acid, 2,6-di(N-phenylcarbamoyl)phenylboronic acid, 3,5-di(N-phenylcarbamoyl)phenylboronic acid, 3,4-di(N-phenylcarbamoyl)phenylboronic acid,
2-(dimethylcarbamoyl)phenylboronic acid, 3-(dimethylcarbamoyl)phenylboronic acid, 4-(dimethylcarbamoyl)phenylboronic acid, 2,3-di(dimethylcarbamoyl)phenylboronic acid, 2,4-di(dimethylcarbamoyl)phenylboronic acid, 2,5-di(dimethylcarbamoyl)phenylboronic acid, 2,6-di(dimethylcarbamoyl)phenylboronic acid, 3,5-di(dimethylcarbamoyl)phenylboronic acid, 3,4-di(dimethylcarbamoyl)phenylboronic acid,
2-succinamidephenylboronic acid, 3-succinamidephenylboronic acid, 4-succinamidephenylboronic acid, 2,3-disuccinamidephenylboronic acid, 2,4-disuccinamidephenylboronic acid, 2,5-disuccinamidephenylboronic acid, 2,6-disuccinamidephenylboronic acid, 3,5-disuccinamidephenylboronic acid, 3,4-disuccinamidephenylboronic acid,
2-phthalimidephenylboronic acid, 3-phthalimidephenylboronic acid, 4-phthalimidephenylboronic acid, 2,3-diphthalimidephenylboronic acid, 2,4-diphthalimidephenylboronic acid, 2,5-diphthalimidephenylboronic acid, 2,6-diphthalimidephenylboronic acid, 3,5-diphthalimidephenylboronic acid, 3,4-diphthalimidephenylboronic acid,
2-thiopheneboronic acid, 3-thiopheneboronic acid, 3-formyl2-thiopheneboronic acid, 2-formyl3-thiopheneboronic acid, 5-formyl2-thiopheneboronic acid, 3-methyl-2-thiopheneboronic acid, 2-methyl-3-thiopheneboronic acid, 3-aceto-2-thiopheneboronic acid, 2-aceto3-thiopheneboronic acid, 5-aceto-2-thiopheneboronic acid,
2-furanboronic acid, 3-furanboronic acid, 2-pyrroleboronic acid, 3-pyrroleboronic acid, 2-imidazoleboronic acid, 4-imidazoleboronic acid, 5-imidazoleboronic acid, 2-pyridineboronic acid, 3-pyridineboronic acid, 4-pyridineboronic acid, 1,-naphthaleneboronic acid, 2-naphthaleneboronic acid, 1,-anthraceneboronic acid, 2-anthraceneboronic acid, 3-anthraceneboronic acid, 4-anthraceneboronic acid, 8-anthraceneboronic acid, 1,-phenanthreneboronic acid, 2-phenanthreneboronic acid, 3-phenanthreneboronic acid, 4-phenanthreneboronic acid, 9-phenanthreneboronic acid, 1,-fluoreneboronic acid, 2-fluoreneboronic acid, 3-fluoreneboronic acid, 4-fluoreneboronic acid, 4-indeneboronic acid, 5-indeneboronic acid, 6-indeneboronic acid, 7-indeneboronic acid, 2-quinolineboronic acid, 3-quinolineboronic acid, 4-quinolineboronic acid, 1,-isoquinolineboronic acid, 3-isoquinolineboronic acid, 4-isoquinolineboronic acid, 5-isoquinolineboronic acid, 6-isoquinolineboronic acid, 7-isoquinolineboronic acid, 8-isoquinolineboronic acid,
3,4-(methylenedioxy)phenylboronic acid, 3,4-(ethylenedioxy)phenylboronic acid, 2,3-(methylenedioxy)phenylboronic acid, 2,3-(ethylenedioxy)phenylboronic acid, 2-biphenylboronic acid, 3-biphenylboronic acid, 4-biphenylboronic acid, 4'-methyl-2-biphenylboronic acid, 4'-methyl-3-biphenylboronic acid, 4'-methyl-4-biphenylboronic acid, 4'-ethyl-2-biphenylboronic acid, 4'-ethyl-3-biphenylboronic acid, 4'-ethyl-4-biphenylboronic acid, and pinacol esters, catechol esters, neopentyl glycol esters and the like of the above-described boronic acids. In the case of boronic acids having an amino group or alkylamino group, these compounds may form salts, for example, together with a mineral acid such as hydrochloric acid, sulfuric acid and the like. The boroxine compound (3) includes compounds obtained by dehydration condensation of the above-described boronic acids, and examples thereof include triphenylboroxin, tri(2-methylphenyl)boroxin, tri(3-methylphenyl)boroxin, tri(4-methylphenyl)boroxin, tri(2-aminophenyl)boroxin, tri(3-aminophenyl)boroxin, tri(4-aminophenyl)boroxin and the like. As the boronic acid and boroxine compounds, these may be used singly, or mixtures of boronic acid and boroxine compounds may be used.

As these organoboron compounds (2) and boroxine compounds (3), commercially available compounds can be used, alternatively, those produced according to methods described in, for example, Chem. Rev., 95, 2457 (1995) and the like can also be used.

The use amount of the organoboron compound (2) when used is usually 0.5 to 10-fold mol, preferably 1 to 3-fold mol based on the aromatic organic compound (1). The use amount of the boroxine compound (3) when used is usually 0.5 to 10-fold mol, preferably 1 to 3-fold mol, in terms of a group represented by Ar² in the formula (3), based on the aromatic organic compound (1).

Examples of the zero-valent nickel catalyst include bis(1,5-cyclooctadiene)nickel(0), tetrakis(triphenyl phosphine)nickel(0), tetracarbonylnickel(0) and the like. Of them, bis(1,5-cyclooctadiene)nickel(0) is preferable. As these zero-valent nickel catalysts, commercially available compounds may be used as they are, or those prepared by any known methods may be used. These may be carried on carries such as activated carbon, silica gel, alumina and the like.

The use amount of the zero-valent nickel catalyst is usually 0.00001 to 1-fold mol, preferably 0.00001 to 0.2-fold mol based on the aromatic organic compound (1).

Examples of the phosphine ligand include triaryl phosphines such as triphenyl phosphine and the like; tricycloalkyl phosphines such as tricyclohexyl phosphine and the like; trialkyl phosphines such as triethyl phosphine, tri-t-butyl phosphine and the like; isocyanated alkyl phosphines such as isocyanated methyl phosphine, isocyanated t-butyl phosphine and the like; isocyanated cycloalkyl phosphines such as isocyanated cyclohexyl phosphine, and the like. Of them, triaryl phosphines, tricycloalkyl phosphines and trialkyl phosphines are preferable, and tricycloalkyl phosphines are more preferable. As these phosphine ligands, commercially available ligands can be usually used. The phosphine ligands may be used singly, or im combination of two or more simultaneously.

The use amount of the phosphine ligand is usually 0.1 to 10-fold mol, preferably 0.5 to 5-fold mo, more preferably 1 to 3-fold mol based on the zero-valent nickel catalyst. In the case of use of a zero-valent nickel catalyst having a phosphine ligand, the use amount of the phosphine ligand may be appropriately determined considering the amount of the phosphine ligand contained in the zero-valent nickel catalyst. For example, in the case of use of tetrakis(triphenyl phosphine)nickel(0), a phosphine ligand (triphenyl phosphine) can be regarded to be used in an amount of 4-fold mol based on the zero-valent nickel catalyst, without separately using a phosphine ligand.

Examples of the base include alkali metal hydroxides sicj as sodium hydroxide, potassium hydroxide and the like; alkaline earth metal hydroxides such as barium hydroxide and the like; alkali metal carbonates such as lithium carbonate, sodium carbonate, potassium carbonate, cesium carbonate and the like; alkaline earth metal carbonates such as calcium carbonate, barium carbonate and the like; alkali metal carboxylates such as sodium acetate, potassium acetate and the like; alkaline earth metal carboxylates such calcium acetate, barium acetate and the like; alkali metal hydrogencarbonates such as sodium hydrogen carbonate, potassium hydrogen carbonate and the like; alkaline earth metal hydrogencarbonates such as calcium hydrogen carbonate, barium hydrogen carbonate and the like; alkali metal phosphates such as tri-lithium phosphate, tri-sodium phosphate, tri-potassium phosphate and the like; alkali metal fluorides such as sodium fluoride, potassium fluoride, cesium fluoride and the like; tertiary amines such as trimethylamine, triethylamine, tributylamine, N,N-dimethylbenzylamine, N,N-diethylaniline, and the like. Of them, alkali metal phosphates are preferable, and tri-potassium phosphate is more preferable. As these bases, commercially available bases can be usually used. The bases may be used singly, or in combination of two or more simultaneously.

The use amount of the base is usually 0.1 to 20-fold mol, preferably 1 to 5-fold mol based on the aromatic organic compound (1).

The production method of the present invention is usually carried out in the presence of a reaction solvent. Examples of the reaction solvent include alcohol solvents such as methanol, ethanol; aprotic solvents such as N-methylpyrrolidone, N,N-dimethylformamide, dimethyl sulfoxide, acetonitrile and the like; ether solvents such as diethyl ether, diisopropyl ether, diethylene glycol dimethyl ether, 1,4-dioxane, tetrahydrofuran and the like; aromatic hydrocarbon solvents such as benzene, toluene, xylene, trifluorotoluene and the like; aliphatic hydrocarbon solvents such as hexane, heptanes and the like. Of them, ether solvents such as diethyl ether, diisopropyl ether, diethylene glycol dimethyl ether, 1,4-dioxane, tetrahydrofuran and the like are preferable, and 1,4-dioxane and tetrahydrofuran are more preferable. These solvents may be used each singly, or in combination of two or more simultaneously.

The use amount of the reaction solvent is usually 0.5 to 200-fold weight based on the aromatic organic compound (1).

The reaction of the present invention may be carried out in the presence of water in the case of use of the aromatic organoboron compound (2). The use amount of water is usually 0 to 2-fold mol based on the aromatic organoboron compound (2). Also in the case of use of the boroxine compound (3), the reaction is preferably carried out in the presence of water. The use amount of water is usually 1 to 3-fold mol in terms of a group represented by Ar² in the formula (3).

The reaction temperature is usually 0 °C to 150 °C, preferably 10 °C to 100 °C.

The mixing order of the at least one compound selected from the group consisting of aromatic organoboron compounds (2) and boroxine compounds (3), the aromatic organic compound (1), the zero-valent nickel catalyst, the phosphine ligand and the base is not particularly restricted.

After completion of the reaction, if necessary, a usual post-treatment operation such as a filtration treatment, liquid partitioning treatment, condensation treatment or the like can be performed on the reaction mixture to isolate a biaryl compound of the formula (4) (hereinafter, referred to as biaryl compound (4)). The resultant biaryl compound (4) may be further purified, for example, by effecting a usual purification treatment such as column chromatography, rectification treatment and the like.

Specific examples of the biaryl compound (4) obtained by the present invention include the following compounds.
2-aminobiphenyl, 3-aminobiphenyl, 4-aminobiphenyl, 2,2'-diaminobiphenyl, 2,3'-diaminobiphenyl, 2,4'-diaminobiphenyl, 3,3'-diaminobiphenyl, 3,4'-diaminobiphenyl, 4,4'-diaminobiphenyl,
2-amino-3-methylbiphenyl, 2-amino-4-methylbiphenyl, 2-amino-5-methylbiphenyl, 2-amino-6-methylbiphenyl, 2-amino-2'-methylbiphenyl, 2-amino-3'-methylbiphenyl, 2-amino-4'-methylbiphenyl, 3-amino-2-methylbiphenyl, 3-amino-4-methylbiphenyl, 3-amino-5-methylbiphenyl, 3-amino-6-methylbiphenyl, 3-amino-2'-methylbiphenyl, 3-amino-3'-methylbiphenyl, 3-amino-4'-methylbiphenyl, 4-amino-2-methylbiphenyl, 4-amino-3-methylbiphenyl, 4-amino-2'-methylbiphenyl, 4-amino-3'-methylbiphenyl, 4-amino-4'-methylbiphenyl,
2,2'-diamino-3-methylbiphenyl, 2,2'-diamino-4-methylbiphenyl, 2,2'-diamino-5-methylbiphenyl, 2,2'-diamino-6-methylbiphenyl, 2,3'-diamino-2-methylbiphenyl, 2,3'-diamino-3-methylbiphenyl, 2,3'-diamino-4-methylbiphenyl, 2,3'-diamino-5-methylbiphenyl, 2,3'-diamino-6-methylbiphenyl, 2,3'-diamino-2'-methylbiphenyl, 2,3'-diamino-4'-methylbiphenyl, 2,3'-diamino-5'-methylbiphenyl, 2,3'-diamino-6'-methylbiphenyl, 2,4'-diamino-3-methylbiphenyl, 2,4'-diamino-4-methylbiphenyl, 2,4'-diamino-5-methylbiphenyl, 2,4'-diamino-6-methylbiphenyl, 2,4'-diamino-2'-methylbiphenyl, 2,4'-diamino-3'-methylbiphenyl, 3,3'-diamino-2-methylbiphenyl, 3,3'-diamino-4-methylbiphenyl, 3,3'-diamino-5-methylbiphenyl, 3,3'7'-diamino-6-methylbiphenyl, 3,4'-diamino-2-methylbiphenyl, 3,4'-diamino-4-methylbiphenyl, 3,4'-diamino-5-methylbiphenyl, 3,4'-diamino-6-methylbiphenyl, 3,4'-diamino-2'-methylbiphenyl, 3,4'-diamino-3'-methylbiphenyl, 4,4'-diamino-2-methylbiphenyl, 4,4'-diamino-3-methylbiphenyl,
2-amino-3-fluorobiphenyl, 2-amino-4-fluorobiphenyl, 2-amino-5-fluorobiphenyl, 2-amino-6-fluorobiphenyl, 2-amino-2'-fluorobiphenyl, 2-amino-3'-fluorobiphenyl, 2-amino-4'-fluorobiphenyl, 3-amino-2-fluorobiphenyl, 3-amino-4-fluorobiphenyl, 3-amino-5-fluorobiphenyl, 3-amino-6-fluorobiphenyl, 3-amino-2'-fluorobiphenyl, 3-amino-3'-fluorobiphenyl, 3-amino-4'-fluorobiphenyl, 4-amino-2-fluorobiphenyl, 4-amino-3-fluorobiphenyl, 4-amino-2'-fluorobiphenyl, 4-amino-3'-fluorobiphenyl, 4-amino-4'-fluorobiphenyl,
2,2'-diamino-3-fluorobiphenyl, 2,2'-diamino-4-fluorobiphenyl, 2,2'-diamino-5-fluorobiphenyl, 2,2'-diamino-6-fluorobiphenyl, 2,3'-diamino-2-fluorobiphenyl, 2,3'-diamino-3-fluorobiphenyl, 2,3'-diamino-4-fluorobiphenyl, 2,3'-diamino-5-fluorobiphenyl, 2,3'-diamino-6-fluorobiphenyl, 2,3'-diamino-2'-fluorobiphenyl, 2,3'-diamino-4'-fluorobiphenyl, 2,3'-diamino-5'-fluorobiphenyl, 2,3'-diamino-6'-fluorobiphenyl, 2,4'-diamino-3-fluorobiphenyl, 2,4'-diamino-4-fluorobiphenyl, 2,4'-diamino-5-fluorobiphenyl, 2,4'-diamino-6-fluorobiphenyl, 2,4'-diamino-2'-fluorobiphenyl, 2,4'-diamino-3'-fluorobiphenyl, 3,3'-diamino-2-fluorobiphenyl, 3,3'-diamino-4-fluorobiphenyl, 3,3'-diamino-5-fluorobiphenyl, 3,3'-diamino-6-fluorobiphenyl, 3,4'-diamino-2-fluorobiphenyl, 3,4'-diamino-4-fluorobiphenyl, 3,4'-diamino-5-fluorobiphenyl, 3,4'-diamino-6-fluorobiphenyl, 3,4'-diamino-2'-fluorobiphenyl, 3,4'-diamino-3'-fluorobiphenyl, 4,4'-diamino-2-fluorobiphenyl, 4,4'-diamino-3-fluorobiphenyl,
2-amino-3-trifluoromethylbiphenyl, 2-amino-4-trifluoromethylbiphenyl, 2-amino-5-trifluoromethylbiphenyl, 2-amino-6-trifluoromethylbiphenyl, 2-amino-2'-trifluoromethylbiphenyl, 2-amino-3'-trifluoromethylbiphenyl, 2-amino-4'-trifluoromethylbiphenyl, 3-amino-2-trifluoromethylbiphenyl, 3-amino-4-trifluoromethylbiphenyl, 3-amino-5-trifluoromethylbiphenyl, 3-amino-6-trifluoromethylbiphenyl, 3-amino-2'-trifluoromethylbiphenyl, 3-amino-3'-trifluoromethylbiphenyl, 3-amino-4'-trifluoromethylbiphenyl, 4-amino-2-trifluoromethylbiphenyl, 4-amino-3-trifluoromethylbiphenyl, 4-amino-2'-trifluoromethylbiphenyl, 4-amino-3'-trifluoromethylbiphenyl, 4-amino-4'-trifluoromethylbiphenyl,
2,2'-diamino-3-trifluoromethylbiphenyl, 2,2'-diamino-4-trifluoromethylbiphenyl, 2,2'-diamino-5-trifluoromethylbiphenyl, 2,2'-diamino-6-trifluoromethylbiphenyl, 2,3'-diamino-2-trifluoromethylbiphenyl, 2,3'-diamino-3-trifluoromethylbiphenyl, 2,3'-diamino-4-trifluoromethylbiphenyl, 2,3'-diamino-5-trifluoromethylbiphenyl, 2,3'-diamino-6-trifluoromethylbiphenyl, 2,3'-diamino-2'-trifluoromethylbiphenyl, 2,3'-diamino-4'-trifluoromethylbiphenyl, 2,3'-diamino-5'-trifluoromethylbiphenyl, 2,3'-diamino-6'-trifluoromethylbiphenyl, 2,4'-diamino-3-trifluoromethylbiphenyl, 2,4'-diamino-4-trifluoromethylbiphenyl, 2,4'-diamino-5-trifluoromethylbiphenyl, 2,4'-diamino-6-trifluoromethylbiphenyl, 2,4'-diamino-2'-trifluoromethylbiphenyl, 2,4'-diamino-3'-trifluoromethylbiphenyl, 3,3'-diamino-2-trifluoromethylbiphenyl, 3,3'-diamino-4-trifluoromethylbiphenyl, 3,3'-diamino-5-trifluoromethylbiphenyl, 3,3'-diamino-6-trifluoromethylbiphenyl, 3,4'-diamino-2-trifluoromethylbiphenyl, 3,4'-diamino-4-trifluoromethylbiphenyl, 3,4'-diamino-5-trifluoromethylbiphenyl, 3,4'-diamino-6-trifluoromethylbiphenyl, 3,4'-diamino-2'-trifluoromethylbiphenyl, 3,4'-diamino-3'-trifluoromethylbiphenyl, 4,4'-diamino-2-trifluoromethylbiphenyl, 4,4'-diamino-3-trifluoromethylbiphenyl, 2-amino-3-methoxybiphenyl, 2-amino-4-methoxybiphenyl, 2-amino-5-methoxybiphenyl, 2-amino-6-methoxybiphenyl, 2-amino-2'-methoxybiphenyl, 2-amino-3'-methoxybiphenyl, 2-amino-4'-methoxybiphenyl, 3-amino-2-methoxybiphenyl, 3-amino-4-methoxybiphenyl, 3-amino-5-methoxybiphenyl, 3-amino-6-methoxybiphenyl, 3-amino-2'-methoxybiphenyl, 3-amino-3'-methoxybiphenyl, 3-amino-4'-methoxybiphenyl, 4-amino-2-methoxybiphenyl, 4-amino-3-methoxybiphenyl, 4-amino-2'-methoxybiphenyl, 4-amino-3'-methoxybiphenyl, 4-amino-4'-methoxybiphenyl,
2,2'-diamino-3-methoxybiphenyl, 2,2'-diamino-4-methoxybiphenyl, 2,2'-diamino-5-methoxybiphenyl, 2,2'-diamino-6-methoxybiphenyl, 2,3'-diamino-2-methoxybiphenyl, 2,3'-diamino-3-methoxybiphenyl, 2,3'-diamino-4-methoxybiphenyl, 2,3'-diamino-5-methoxybiphenyl, 2,3'-diamino-6-methoxybiphenyl, 2,3'-diamino-2'-methoxybiphenyl, 2,3'-diamino-4'-methoxybiphenyl, 2,3'-diamino-5'-methoxybiphenyl, 2,3'-diamino-6'-methoxybiphenyl, 2,4'-diamino-3-methoxybiphenyl, 2,4'-diamino-4-methoxybiphenyl, 2,4'-diamino-5-methoxybiphenyl, 2,4'-diamino-6-methoxybiphenyl, 2,4'-diamino-2'-methoxybiphenyl, 2,4'-diamino-3'-methoxybiphenyl, 3,3'-diamino-2-methoxybiphenyl, 3,3'-diamino-4-methoxybiphenyl, 3,3'-diamino-5-methoxybiphenyl, 3,3'-diamino-6-methoxybiphenyl, 3,4'-diamino-2-methoxybiphenyl, 3,4'-diamino-4-methoxybiphenyl, 3,4'-diamino-5-methoxybiphenyl, 3,4'-diamino-6-methoxybiphenyl, 3,4'-diamino-2'-methoxybiphenyl, 3,4'-diamino-3'-methoxybiphenyl, 4,4'-diamino-2-methoxybiphenyl, 4,4'-diamino-3-methoxybiphenyl, 2-amino-3-ethoxybiphenyl, 2-amino-4-ethoxybiphenyl, 2-amino-5-ethoxybiphenyl, 2-amino-6-ethoxybiphenyl, 2-amino-2'-ethoxybiphenyl, 2-amino-3'-ethoxybiphenyl, 2-amino-4'-ethoxybiphenyl, 3-amino-2-ethoxybiphenyl, 3-amino-4-ethoxybiphenyl, 3-amino-5-ethoxybiphenyl, 3-amino-6-ethoxybiphenyl, 3-amino-2'-ethoxybiphenyl, 3-amino-3'-ethoxybiphenyl, 3-amino-4'-ethoxybiphenyl, 4-amino-2-ethoxybiphenyl, 4-amino-3-ethoxybiphenyl, 4-amino-2'-ethoxybiphenyl, 4-amino-3'-ethoxybiphenyl, 4-amino-4'-ethoxybiphenyl,
2,2'-diamino-3-ethoxybiphenyl, 2,2'-diamino-4-ethoxybiphenyl, 2,2'-diamino-5-ethoxybiphenyl, 2,2'-diamino-6-ethoxybiphenyl, 2,3'-diamino-2-ethoxybiphenyl, 2,3'-diamino-3-ethoxybiphenyl, 2,3'-diamino-4-ethoxybiphenyl, 2,3'-diamino-5-ethoxybiphenyl, 2,3'-diamino-6-ethoxybiphenyl, 2,3'-diamino-2'-ethoxybiphenyl, 2,3'-diamino-4'-ethoxybiphenyl, 2,3'-diamino-5'-ethoxybiphenyl, 2,3'-diamino-6'-ethoxybiphenyl, 2,4'-diamino-3-ethoxybiphenyl, 2,4'-diamino-4-ethoxybiphenyl, 2,4'-diamino-5-ethoxybiphenyl, 2,4'-diamino-6-ethoxybiphenyl, 2,4'-diamino-2'-ethoxybiphenyl, 2,4'-diamino-3'-ethoxybiphenyl, 3,3'-diamino-2-ethoxybiphenyl, 3,3'-diamino-4-ethoxybiphenyl, 3,3'-diamino-5-ethoxybiphenyl, 3,3'-diamino-6-ethoxybiphenyl, 3,4'-diamino-2-ethoxybiphenyl, 3,4'-diamino-4-ethoxybiphenyl, 3,4'-diamino-5-ethoxybiphenyl, 3,4'-diamino-6-ethoxybiphenyl, 3,4'-diamino-2'-ethoxybiphenyl, 3,4'-diamino-3'-ethoxybiphenyl, 4,4'-diamino-2-ethoxybiphenyl, 4,4'-diamino-3-ethoxybiphenyl,
2-amino-3-phenoxybiphenyl, 2-amino-4-phenoxybiphenyl, 2-amino-5-phenoxybiphenyl, 2-amino-6-phenoxybiphenyl, 2-amino-2'-phenoxybiphenyl, 2-amino-3'-phenoxybiphenyl, 2-amino-4'-phenoxybiphenyl, 3-amino-2-phenoxybiphenyl, 3-amino-4-phenoxybiphenyl, 3-amino-5-phenoxybiphenyl, 3-amino-6-phenoxybiphenyl, 3-amino-2'-phenoxybiphenyl, 3-amino-3'-phenoxybiphenyl, 3-amino-4'-phenoxybiphenyl, 4-amino-2-phenoxybiphenyl, 4-amino-3-phenoxybiphenyl, 4-amino-2'-phenoxybiphenyl, 4-amino-3'-phenoxybiphenyl, 4-amino-4'-phenoxybiphenyl,
2,2'-diamino-3-phenoxybiphenyl, 2,2'-diamino-4-phenoxybiphenyl, 2,2'-diamino-5-phenoxybiphenyl, 2,2'-diamino-6-phenoxybiphenyl, 2,3'-diamino-2-phenoxybiphenyl, 2,3'-diamino-3-phenoxybiphenyl, 2,3'-diamino-4-phenoxybiphenyl, 2,3'-diamino-5-phenoxybiphenyl, 2,3'-diamino-6-phenoxybiphenyl, 2,3'-diamino-2'-phenoxybiphenyl, 2,3'-diamino-4'-phenoxybiphenyl, 2,3'-diamino-5'-phenoxybiphenyl, 2,3'-diamino-6'-phenoxybiphenyl, 2,4'-diamino-3-phenoxybiphenyl, 2,4'-diamino-4-phenoxybiphenyl, 2,4'-diamino-5-phenoxybiphenyl, 2,4'-diamino-6-phenoxybiphenyl, 2,4'-diamino-2'-phenoxybiphenyl, 2,4'-diamino-3'-phenoxybiphenyl, 3,3'-diamino-2-phenoxybiphenyl, 3,3'-diamino-4-phenoxybiphenyl, 3,3'-diamino-5-phenoxybiphenyl, 3,3'-diamino-6-phenoxybiphenyl, 3,4'-diamino-2-phenoxybiphenyl, 3,4'-diamino-4-phenoxybiphenyl, 3,4'-diamino-5-phenoxybiphenyl, 3,4'-diamino-6-phenoxybiphenyl, 3,4'-diamino-2'-phenoxybiphenyl, 3,4'-diamino-3'-phenoxybiphenyl, 4,4'-diamino-2-phenoxybiphenyl, 4,4'-diamino-3-phenoxybiphenyl,
2-amino-3-benzyloxybiphenyl, 2-amino-4-benzyloxybiphenyl, 2-amino-5-benzyloxybiphenyl, 2-amino-6-benzyloxybiphenyl, 2-amino-2'-benzyloxybiphenyl, 2-amino-3'-benzyloxybiphenyl, 2-amino-4'-benzyloxybiphenyl, 3-amino-2-benzyloxybiphenyl, 3-amino-4-benzyloxybiphenyl, 3-amino-5-benzyloxybiphenyl, 3-amino-6-benzyloxybiphenyl, 3-amino-2'-benzyloxybiphenyl, 3-amino-3'-benzyloxybiphenyl, 3-amino-4'-benzyloxybiphenyl, 4-amino-2-benzyloxybiphenyl, 4-amino-3-benzyloxybiphenyl, 4-amino-2'-benzyloxybiphenyl, 4-amino-3'-benzyloxybiphenyl, 4-amino-4'-benzyloxybiphenyl,
2,2'-diamino-3-benzyloxybiphenyl, 2,2'-diamino-4-benzyloxybiphenyl, 2,2'-diamino-5-benzyloxybiphenyl, 2,2'-diamino-6-benzyloxybiphenyl, 2,3'-diamino-2-benzyloxybiphenyl, 2,3'-diamino-3-benzyloxybiphenyl, 2,3'-diamino-4-benzyloxybiphenyl, 2,3'-diamino-5-benzyloxybiphenyl, 2,3'-diamino-6-benzyloxybiphenyl, 2,3'-diamino-2'-benzyloxybiphenyl, 2,3'-diamino-4'-benzyloxybiphenyl, 2,3'-diamino-5'-benzyloxybiphenyl, 2,3'-diamino-6'-benzyloxybiphenyl, 2,4'-diamino-3-benzyloxybiphenyl, 2,4'-diamino-4-benzyloxybiphenyl, 2,4'-diamino-5-benzyloxybiphenyl, 2,4'-diamino-6-benzyloxybiphenyl, 2,4'-diamino-2'-benzyloxybiphenyl, 2,4'-diamino-3'-benzyloxybiphenyl, 3,3'-diamino-2-benzyloxybiphenyl, 3,3'-diamino-4-benzyloxybiphenyl, 3,3'-diamino-5-benzyloxybiphenyl, 3,3'-diamino-6-benzyloxybiphenyl, 3,4'-diamino-2-benzyloxybiphenyl, 3,4'-diamino-4-benzyloxybiphenyl, 3,4'-diamino-5-benzyloxybiphenyl, 3,4'-diamino-6-benzyloxybiphenyl, 3,4'-diamino-2'-benzyloxybiphenyl, 3,4'-diamino-3'-benzyloxybiphenyl, 4,4'-diamino-2-benzyloxybiphenyl, 4,4'-diamino-3-benzyloxybiphenyl,
2-amino-3-methylthiobiphenyl, 2-amino-4-methylthiobiphenyl, 2-amino-5-methylthiobiphenyl, 2-amino-6-methylthiobiphenyl, 2-amino-2'-methylthiobiphenyl, 2-amino-3'-methylthiobiphenyl, 2-amino-4'-methylthiobiphenyl, 3-amino-2-methylthiobiphenyl, 3-amino-4-methylthiobiphenyl, 3-amino-5-methylthiobiphenyl, 3-amino-6-methylthiobiphenyl, 3-amino-2'-methylthiobiphenyl, 3-amino-3'-methylthiobiphenyl, 3-amino-4'-methylthiobiphenyl, 4-amino-2-methylthiobiphenyl, 4-amino-3-methylthiobiphenyl, 4-amino-2'-methylthiobiphenyl, 4-amino-3'-methylthiobiphenyl, 4-amino-4'-methylthiobiphenyl,
2,2'-diamino-3-methylthiobiphenyl, 2,2'-diamino-4-methylthiobiphenyl, 2,2'-diamino-5-methylthiobiphenyl, 2,2'-diamino-6-methylthiobiphenyl, 2,3'-diamino-2-methylthiobiphenyl, 2,3'-diamino-3-methylthiobiphenyl, 2,3'-diamino-4-methylthiobiphenyl, 2,3'-diamino-5-methylthiobiphenyl, 2,3'-diamino-6-methylthiobiphenyl, 2,3'-diamino-2'-methylthiobiphenyl, 2,3'-diamino-4'-methylthiobiphenyl, 2,3'-diamino-5'-methylthiobiphenyl, 2,3'-diamino-6'-methylthiobiphenyl, 2,4'-diamino-3-methylthiobiphenyl, 2,4'-diamino-4-methylthiobiphenyl, 2,4'-diamino-5-methylthiobiphenyl, 2,4'-diamino-6-methylthiobiphenyl, 2,4'-diamino-2'-methylthiobiphenyl, 2,4'-diamino-3'-methylthiobiphenyl, 3,3'-diamino-2-methylthiobiphenyl, 3,3'-diamino-4-methylthiobiphenyl, 3,3'-diamino-5-methylthiobiphenyl, 3,3'-diamino-6-methylthiobiphenyl, 3,4'-diamino-2-methylthiobiphenyl, 3,4'-diamino-4-methylthiobiphenyl, 3,4'-diamino-5-methylthiobiphenyl, 3,4'-diamino-6-methylthiobiphenyl, 3,4'-diamino-2'-methylthiobiphenyl, 3,4'-diamino-3'-methylthiobiphenyl, 4,4'-diamino-2-methylthiobiphenyl, 4,4'-diamino-3-methylthiobiphenyl
2-amino-3-ethylthiobiphenyl, 2-amino-4-ethylthiobiphenyl, 2-amino-5-ethylthiobiphenyl, 2-amino-6-ethylthiobiphenyl, 2-amino-2'-ethylthiobiphenyl, 2-amino-3'-ethylthiobiphenyl, 2-amino-4'-ethylthiobiphenyl, 3-amino-2-ethylthiobiphenyl, 3-amino-4-ethylthiobiphenyl, 3-amino-5-ethylthiobiphenyl, 3-amino-6-ethylthiobiphenyl, 3-amino-2'-ethylthiobiphenyl, 3-amino-3'-ethylthiobiphenyl, 3-amino-4'-ethylthiobiphenyl, 4-amino-2-ethylthiobiphenyl, 4-amino-3-ethylthiobiphenyl, 4-amino-2'-ethylthiobiphenyl, 4-amino-3'-ethylthiobiphenyl, 4-amino-4'-ethylthiobiphenyl,
2,2'-diamino-3-ethylthiobiphenyl, 2,2'-diamino-4-ethylthiobiphenyl, 2,2'-diamino-5-ethylthiobiphenyl, 2,2'-diamino-6-ethylthiobiphenyl, 2,3'-diamino-2-ethylthiobiphenyl, 2,3'-diamino-3-ethylthiobiphenyl, 2,3'-diamino-4-ethylthiobiphenyl, 2,3'-diamino-5-ethylthiobiphenyl, 2,3'-diamino-6-ethylthiobiphenyl, 2,3'-diamino-2'-ethylthiobiphenyl, 2,3'-diamino-4'-ethylthiobiphenyl, 2,3'-diamino-5'-ethylthiobiphenyl, 2,3'-diamino-6'-ethylthiobiphenyl, 2,4'-diamino-3-ethylthiobiphenyl, 2,4'-diamino-4-ethylthiobiphenyl, 2,4'-diamino-5-ethylthiobiphenyl, 2,4'-diamino-6-ethylthiobiphenyl, 2,4'-diamino-2'-ethylthiobiphenyl, 2,4'-diamino-3'-ethylthiobiphenyl, 3,3'-diamino-2-ethylthiobiphenyl, 3,3'-diamino-4-ethylthiobiphenyl, 3,3'-diamino-5-ethylthiobiphenyl, 3,3'-diamino-6-ethylthiobiphenyl, 3,4'-diamino-2-ethylthiobiphenyl, 3,4'-diamino-4-ethylthiobiphenyl, 3,4'-diamino-5-ethylthiobiphenyl, 3,4'-diamino-6-ethylthiobiphenyl, 3,4'-diamino-2'-ethylthiobiphenyl, 3,4'-diamino-3'-ethylthiobiphenyl, 4,4'-diamino-2-ethylthiobiphenyl, 4,4'-diamino-3-ethylthiobiphenyl
2-amino-3-phenylthiobiphenyl, 2-amino-4-phenylthiobiphenyl, 2-amino-5-phenylthiobiphenyl, 2-amino-6-phenylthiobiphenyl, 2-amino-2'-phenylthiobiphenyl, 2-amino-3'-phenylthiobiphenyl, 2-amino-4'-phenylthiobiphenyl, 3-amino-2-phenylthiobiphenyl, 3-amino-4-phenylthiobiphenyl, 3-amino-5-phenylthiobiphenyl, 3-amino-6-phenylthiobiphenyl, 3-amino-2'-phenylthiobiphenyl, 3-amino-3'-phenylthiobiphenyl, 3-amino-4'-phenylthiobiphenyl, 4-amino-2-phenylthiobiphenyl, 4-amino-3-phenylthiobiphenyl, 4-amino-2'-phenylthiobiphenyl, 4-amino-3'-phenylthiobiphenyl, 4-amino-4'-phenylthiobiphenyl,
2,2'-diamino-3-phenylthiobiphenyl, 2,2'-diamino-4-phenylthiobiphenyl, 2,2'-diamino-5-phenylthiobiphenyl, 2,2'-diamino-6-phenylthiobiphenyl, 2,3'-diamino-2-phenylthiobiphenyl, 2,3'-diamino-3-phenylthiobiphenyl, 2,3'-diamino-4-phenylthiobiphenyl, 2,3'-diamino-5-phenylthiobiphenyl, 2,3'-diamino-6-phenylthiobiphenyl, 2,3'-diamino-2'-phenylthiobiphenyl, 2,3'-diamino-4'-phenylthiobiphenyl, 2,3'-diamino-5'-phenylthiobiphenyl, 2,3'-diamino-6'-phenylthiobiphenyl, 2,4'-diamino-3-phenylthiobiphenyl, 2,4'-diamino-4-phenylthiobiphenyl, 2,4'-diamino-5-phenylthiobiphenyl, 2,4'-diamino-6-phenylthiobiphenyl, 2,4'-diamino-2'-phenylthiobiphenyl, 2,4'-diamino-3'-phenylthiobiphenyl, 3,3'-diamino-2-phenylthiobiphenyl, 3,3'-diamino-4-phenylthiobiphenyl, 3,3'-diamino-5-phenylthiobiphenyl, 3,3'-diamino-6-phenylthiobiphenyl, 3,4'-diamino-2-phenylthiobiphenyl, 3,4'-diamino-4-phenylthiobiphenyl, 3,4'-diamino-5-phenylthiobiphenyl, 3,4'-diamino-6-phenylthiobiphenyl, 3,4'-diamino-2'-phenylthiobiphenyl, 3,4'-diamino-3'-phenylthiobiphenyl, 4,4'-diamino-2-phenylthiobiphenyl, 4,4'-diamino-3-phenylthiobiphenyl
2-amino-3-methylaminobiphenyl, 2-amino-4-methylaminobiphenyl, 2-amino-5-methylaminobiphenyl, 2-amino-6-methylaminobiphenyl, 2-amino-2'-methylaminobiphenyl, 2-amino-3'-methylaminobiphenyl, 2-amino-4'-methylaminobiphenyl, 3-amino-2-methylaminobiphenyl, 3-amino-4-methylaminobiphenyl, 3-amino-5-methylaminobiphenyl, 3-amino-6-methylaminobiphenyl, 3-amino-2'-methylaminobiphenyl, 3-amino-3'-methylaminobiphenyl, 3-amino-4'-methylaminobiphenyl, 4-amino-2-methylaminobiphenyl, 4-amino-3-methylaminobiphenyl, 4-amino-2'-methylaminobiphenyl, 4-amino-3'-methylaminobiphenyl, 4-amino-4'-methylaminobiphenyl,
2,2'-diamino-3-methylaminobiphenyl, 2,2'-diamino-4-methylaminobiphenyl, 2,2'-diamino-5-methylaminobiphenyl, 2,2'-diamino-6-methylaminobiphenyl, 2,3'-diamino-2-methylaminobiphenyl, 2,3'-diamino-3-methylaminobiphenyl, 2,3'-diamino-4-methylaminobiphenyl, 2,3'-diamino-5-methylaminobiphenyl, 2,3'-diamino-6-methylaminobiphenyl, 2,3'-diamino-2'-methylaminobiphenyl, 2,3'-diamino-4'-methylaminobiphenyl, 2,3'-diamino-5'-methylaminobiphenyl, 2,3'-diamino-6'-methylaminobiphenyl, 2,4'-diamino-3-methylaminobiphenyl, 2,4'-diamino-4-methylaminobiphenyl, 2,4'-diamino-5-methylaminobiphenyl, 2,4'-diamino-6-methylaminobiphenyl, 2,4'-diamino-2'-methylaminobiphenyl, 2,4'-diamino-3'-methylaminobiphenyl, 3,3'-diamino-2-methylaminobiphenyl, 3,3'-diamino-4-methylaminobiphenyl, 3,3'-diamino-5-methylaminobiphenyl, 3,3'-diamino-6-methylaminobiphenyl, 3,4'-diamino-2-methylaminobiphenyl, 3,4'-diamino-4-methylaminobiphenyl, 3,4'-diamino-5-methylaminobiphenyl, 3,4'-diamino-6-methylaminobiphenyl, 3,4'-diamino-2'-methylaminobiphenyl, 3,4'-diamino-3'-methylaminobiphenyl, 4,4'-diamino-2-methylaminobiphenyl, 4,4'-diamino-3-methylaminobiphenyl
2-amino-3-dimethylaminobiphenyl, 2-amino-4-dimethylaminobiphenyl, 2-amino-5-dimethylaminobiphenyl, 2-amino-6-dimethylaminobiphenyl, 2-amino-2'-dimethylaminobiphenyl, 2-amino-3'-dimethylaminobiphenyl, 2-amino-4'-dimethylaminobiphenyl, 3-amino-2-dimethylaminobiphenyl, 3-amino-4-dimethylaminobiphenyl, 3-amino-5-dimethylaminobiphenyl, 3-amino-6-dimethylaminobiphenyl, 3-amino-2'-dimethylaminobiphenyl, 3-amino-3'-dimethylaminobiphenyl, 3-amino-4'-dimethylaminobiphenyl, 4-amino-2-dimethylaminobiphenyl, 4-amino-3-dimethylaminobiphenyl, 4-amino-2'-dimethylaminobiphenyl, 4-amino-3'-dimethylaminobiphenyl, 4-amino-4'-dimethylaminobiphenyl,
2,2'-diamino-3-dimethylaminobiphenyl, 2,2'-diamino-4-dimethylaminobiphenyl, 2,2'-diamino-5-dimethylaminobiphenyl, 2,2'-diamino-6-dimethylaminobiphenyl, 2,3'-diamino-2-dimethylaminobiphenyl, 2,3'-diamino-3-dimethylaminobiphenyl, 2,3'-diamino-4-dimethylaminobiphenyl, 2,3'-diamino-5-dimethylaminobiphenyl, 2,3'-diamino-6-dimethylaminobiphenyl, 2,3'-diamino-2'-dimethylaminobiphenyl, 2,3'-diamino-4'-dimethylaminobiphenyl, 2,3'-diamino-5'-dimethylaminobiphenyl, 2,3'-diamino-6'-dimethylaminobiphenyl, 2,4'-diamino-3-dimethylaminobiphenyl, 2,4'-diamino-4-dimethylaminobiphenyl, 2,4'-diamino-5-dimethylaminobiphenyl, 2,4'-diamino-6-dimethylaminobiphenyl, 2,4'-diamino-2'-dimethylaminobiphenyl, 2,4'-diamino-3'-dimethylaminobiphenyl, 3,3'-diamino-2-dimethylaminobiphenyl, 3,3'-diamino-4-dimethylaminobiphenyl, 3,3'-diamino-5-dimethylaminobiphenyl, 3,3'-diamino-6-dimethylaminobiphenyl, 3,4'-diamino-2-dimethylaminobiphenyl, 3,4'-diamino-4-dimethylaminobiphenyl, 3,4'-diamino-5-dimethylaminobiphenyl, 3,4'-diamino-6-dimethylaminobiphenyl, 3,4'-diamino-2'-dimethylaminobiphenyl, 3,4'-diamino-3'-dimethylaminobiphenyl, 4,4'-diamino-2-dimethylaminobiphenyl, 4,4'-diamino-3-dimethylaminobiphenyl
2-amino-3-t-butoxycarbonylaminobiphenyl, 2-amino-4-t-butoxycarbonylaminobiphenyl, 2-amino-5-t-butoxycarbonylaminobiphenyl, 2-amino-6-t-butoxycarbonylaminobiphenyl, 2-amino-2'-t-butoxycarbonylaminobiphenyl, 2-amino-3'-t-butoxycarbonylaminobiphenyl, 2-amino-4'-t-butoxycarbonylaminobiphenyl, 3-amino-2-t-butoxycarbonylaminobiphenyl, 3-amino-4-t-butoxycarbonylaminobiphenyl, 3-amino-5-t-butoxycarbonylaminobiphenyl, 3-amino-6-t-butoxycarbonylaminobiphenyl, 3-amino-2'-t-butoxycarbonylaminobiphenyl, 3-amino-3'-t-butoxycarbonylaminobiphenyl, 3-amino-4'-t-butoxycarbonylaminobiphenyl, 4-amino-2-t-butoxycarbonylaminobiphenyl, 4-amino-3-t-butoxycarbonylaminobiphenyl, 4-amino-2'-t-butoxycarbonylaminobiphenyl, 4-amino-3'-t-butoxycarbonylaminobiphenyl, 4-amino-4'-t-butoxycarbonylaminobiphenyl,
2,2'-diamino-3-t-butoxycarbonylaminobiphenyl, 2,2'-diamino-4-t-butoxycarbonylaminobiphenyl, 2,2'-diamino-5-t-butoxycarbonylaminobiphenyl, 2,2'-diamino-6-t-butoxycarbonylaminobiphenyl, 2,3'-diamino-2-t-butoxycarbonylaminobiphenyl, 2,3'-diamino-3-t-butoxycarbonylaminobiphenyl, 2,3'-diamino-4-t-butoxycarbonylaminobiphenyl, 2,3'-diamino-5-t-butoxycarbonylaminobiphenyl, 2,3'-diamino-6-t-butoxycarbonylaminobiphenyl, 2,3'-diamino-2'-t-butoxycarbonylaminobiphenyl, 2,3'-diamino-4'-t-butoxycarbonylaminobiphenyl, 2,3'-diamino-5'-t-butoxycarbonylaminobiphenyl, 2,3'-diamino-6'-t-butoxycarbonylaminobiphenyl, 2,4'-diamino-3-t-butoxycarbonylaminobiphenyl, 2,4'-diamino-4-t-butoxycarbonylaminobiphenyl, 2,4'-diamino-5-t-butoxycarbonylaminobiphenyl, 2,4'-diamino-6-t-butoxycarbonylaminobiphenyl, 2,4'-diamino-2'-t-butoxycarbonylaminobiphenyl, 2,4'-diamino-3'-t-butoxycarbonylaminobiphenyl, 3,3'-diamino-2-t-butoxycarbonylaminobiphenyl, 3,3'-diamino-4-t-butoxycarbonylaminobiphenyl, 3,3'-diamino-5-t-butoxycarbonylaminobiphenyl, 3,3'-diamino-6-t-butoxycarbonylaminobiphenyl, 3,4'-diamino-2-t-butoxycarbonylaminobiphenyl, 3,4'-diamino-4-t-butoxycarbonylaminobiphenyl, 3,4'-diamino-5-t-butoxycarbonylaminobiphenyl, 3,4'-diamino-6-t-butoxycarbonylaminobiphenyl, 3,4'-diamino-2'-t-butoxycarbonylaminobiphenyl, 3,4'-diamino-3'-t-butoxycarbonylaminobiphenyl, 4,4'-diamino-2-t-butoxycarbonylaminobiphenyl, 4,4'-diamino-3-t-butoxycarbonylaminobiphenyl,

2-amino-3-t-butoxycarbonylaminobiphenyl, 2-amino-4-t-butoxycarbonylaminobiphenyl, 2-amino-5-t-butoxycarbonylaminobiphenyl, 2-amino-6-t-butoxycarbonylaminobiphenyl, 2-amino-2'-t-butoxycarbonylaminobiphenyl, 2-amino-3'-t-butoxycarbonylaminobiphenyl, 2-amino-4'-t-butoxycarbonylaminobiphenyl, 3-amino-2-t-butoxycarbonylaminobiphenyl, 3-amino-4-t-butoxycarbonylaminobiphenyl, 3-amino-5-t-butoxycarbonylaminobiphenyl, 3-amino-6-t-butoxycarbonylaminobiphenyl, 3-amino-2'-t-butoxycarbonylaminobiphenyl, 3-amino-3'-t-butoxycarbonylaminobiphenyl, 3-amino-4'-t-butoxycarbonylaminobiphenyl, 4-amino-2-t-butoxycarbonylaminobiphenyl, 4-amino-3-t-butoxycarbonylaminobiphenyl, 4-amino-2'-t-butoxycarbonylaminobiphenyl, 4-amino-3'-t-butoxycarbonylaminobiphenyl, 4-amino-4'-t-butoxycarbonylaminobiphenyl, 2,2'-diamino-3-t-butoxycarbonylaminobiphenyl, 2,2'-diamino-4-t-butoxycarbonylaminobiphenyl, 2,2'-diamino-5-t-butoxycarbonylaminobiphenyl, 2,2'-diamino-6-t-butoxycarbonylaminobiphenyl, 2,3'-diamino-2-t-butoxycarbonylaminobiphenyl, 2,3'-diamino-3-t-butoxycarbonylaminobiphenyl, 2,3'-diamino-4-t-butoxycarbonylaminobiphenyl, 2,3'-diamino-5-t-butoxycarbonylaminobiphenyl, 2,3'-diamino-6-t-butoxycarbonylaminobiphenyl, 2,3'-diamino-2'-t-butoxycarbonylaminobiphenyl, 2,3'-diamino-4'-t-butoxycarbonylaminobiphenyl, 2,3'-diamino-5'-t-butoxycarbonylaminobiphenyl, 2,3'-diamino-6'-t-butoxycarbonylaminobiphenyl, 2,4'-diamino-3-t-butoxycarbonylaminobiphenyl, 2,4'-diamino-4-t-butoxycarbonylaminobiphenyl, 2,4'-diamino-5-t-butoxycarbonylaminobiphenyl, 2,4'-diamino-6-t-butoxycarbonylaminobiphenyl, 2,4'-diamino-2'-t-butoxycarbonylaminobiphenyl, 2,4'-diamino-3'-t-butoxycarbonylaminobiphenyl, 3,3'-diamino-2-t-butoxycarbonylaminobiphenyl, 3,3'-diamino-4-t-butoxycarbonylaminobiphenyl, 3,3'-diamino-5-t-butoxycarbonylaminobiphenyl, 3,3'-diamino-6-t-butoxycarbonylaminobiphenyl, 3,4'-diamino-2-t-butoxycarbonylaminobiphenyl, 3,4'-diamino-4-t-butoxycarbonylaminobiphenyl, 3,4'-diamino-5-t-butoxycarbonylaminobiphenyl, 3,4'-diamino-6-t-butoxycarbonylaminobiphenyl, 3,4'-diamino-2'-t-butoxycarbonylaminobiphenyl, 3,4'-diamino-3'-t-butoxycarbonylaminobiphenyl, 4,4'-diamino-2-t-butoxycarbonylaminobiphenyl, 4,4'-diamino-3-t-butoxycarbonylaminobiphenyl, 2-amino-3-benzyloxycarbonylaminobiphenyl, 2-amino-4-benzyloxycarbonylaminobiphenyl, 2-amino-5-benzyloxycarbonylaminobiphenyl, 2-amino-6-benzyloxycarbonylaminobiphenyl, 2-amino-2'-benzyloxycarbonylaminobiphenyl, 2-amino-3'-benzyloxycarbonylaminobiphenyl, 2-amino-4'-benzyloxycarbonylaminobiphenyl, 3-amino-2-benzyloxycarbonylaminobiphenyl, 3-amino-4-benzyloxycarbonylaminobiphenyl, 3-amino-5-benzyloxycarbonylaminobiphenyl, 3-amino-6-benzyloxycarbonylaminobiphenyl, 3-amino-2'-benzyloxycarbonylaminobiphenyl, 3-amino-3'-benzyloxycarbonylaminobiphenyl, 3-amino-4'-benzyloxycarbonylaminobiphenyl, 4-amino-2-benzyloxycarbonylaminobiphenyl, 4-amino-3-benzyloxycarbonylaminobiphenyl, 4-amino-2'-benzyloxycarbonylaminobiphenyl, 4-amino-3'-benzyloxycarbonylaminobiphenyl, 4-amino-4'-benzyloxycarbonylaminobiphenyl,
2,2'-diamino-3-benzyloxycarbonylaminobiphenyl, 2,2'-diamino-4-benzyloxycarbonylaminobiphenyl, 2,2'-diamino-5-benzyloxycarbonylaminobiphenyl, 2,2'-diamino-6-benzyloxycarbonylaminobiphenyl, 2,3'-diamino-2-benzyloxycarbonylaminobiphenyl, 2,3'-diamino-3-benzyloxycarbonylaminobiphenyl, 2,3'-diamino-4-benzyloxycarbonylaminobiphenyl, 2,3'-diamino-5-benzyloxycarbonylaminobiphenyl, 2,3'-diamino-6-benzyloxycarbonylaminobiphenyl, 2,3'-diamino-2'-benzyloxycarbonylaminobiphenyl, 2,3'-diamino-4'-benzyloxycarbonylaminobiphenyl, 2,3'-diamino-5'-benzyloxycarbonylaminobiphenyl, 2,3'-diamino-6'-benzyloxycarbonylaminobiphenyl, 2,4'-diamino-3-benzyloxycarbonylaminobiphenyl, 2,4'-diamino-4-benzyloxycarbonylaminobiphenyl, 2,4'-diamino-5-benzyloxycarbonylaminobiphenyl, 2,4'-diamino-6-benzyloxycarbonylaminobiphenyl, 2,4'-diamino-2'-benzyloxycarbonylaminobiphenyl, 2,4'-diamino-3'-benzyloxycarbonylaminobiphenyl, 3,3'-diamino-2-benzyloxycarbonylaminobiphenyl, 3,3'-diamino-4-benzyloxycarbonylaminobiphenyl, 3,3'-diamino-5-benzyloxycarbonylaminobiphenyl, 3,3'-diamino-6-benzyloxycarbonylaminobiphenyl, 3,4'-diamino-2-benzyloxycarbonylaminobiphenyl, 3,4'-diamino-4-benzyloxycarbonylaminobiphenyl, 3,4'-diamino-5-benzyloxycarbonylaminobiphenyl, 3,4'-diamino-6-benzyloxycarbonylaminobiphenyl, 3,4'-diamino-2'-benzyloxycarbonylaminobiphenyl, 3,4'-diamino-3'-benzyloxycarbonylaminobiphenyl, 4,4'-diamino-2-benzyloxycarbonylaminobiphenyl, 4,4'-diamino-3-benzyloxycarbonylaminobiphenyl
2-amino-3-p-methoxyphenoxycarbonylaminobiphenyl, 2-amino-4-p-methoxyphenoxycarbonylaminobiphenyl, 2-amino-5-p-methoxyphenoxycarbonylaminobiphenyl, 2-amino-6-p-methoxyphenoxycarbonylaminobiphenyl, 2-amino-2'-p-methoxyphenoxycarbonylaminobiphenyl, 2-amino-3'-p-methoxyphenoxycarbonylaminobiphenyl, 2-amino-4'-p-methoxyphenoxycarbonylaminobiphenyl, 3-amino-2-p-methoxyphenoxycarbonylaminobiphenyl, 3-amino-4-p-methoxyphenoxycarbonylaminobiphenyl, 3-amino-5-p-methoxyphenoxycarbonylaminobiphenyl, 3-amino-6-p-methoxyphenoxycarbonylaminobiphenyl, 3-amino-2'-p-methoxyphenoxycarbonylaminobiphenyl, 3-amino-3'-p-methoxyphenoxycarbonylaminobiphenyl, 3-amino-4'-p-methoxyphenoxycarbonylaminobiphenyl, 4-amino-2-p-methoxyphenoxycarbonylaminobiphenyl, 4-amino-3-p-methoxyphenoxycarbonylaminobiphenyl, 4-amino-2'-p-methoxyphenoxycarbonylaminobiphenyl, 4-amino-3'-p-methoxyphenoxycarbonylaminobiphenyl, 4-amino-4'-p-methoxyphenoxycarbonylaminobiphenyl,
2,2'-diamino-3-p-methoxyphenoxycarbonylaminobiphenyl, 2,2'-diamino-4-p-methoxyphenoxycarbonylaminobiphenyl, 2,2'-diamino-5-p-methoxyphenoxycarbonylaminobiphenyl, 2,2'-diamino-6-p-methoxyphenoxycarbonylaminobiphenyl, 2,3'-diamino-2-p-methoxyphenoxycarbonylaminobiphenyl, 2,3'-diamino-3-p-methoxyphenoxycarbonylaminobiphenyl, 2,3'-diamino-4-p-methoxyphenoxycarbonylaminobiphenyl, 2,3'-diamino-5-p-methoxyphenoxycarbonylaminobiphenyl, 2,3'-diamino-6-p-methoxyphenoxycarbonylaminobiphenyl, 2,3'-diamino-2'-p-methoxyphenoxycarbonylaminobiphenyl, 2,3'-diamino-4'-p-methoxyphenoxycarbonylaminobiphenyl, 2,3'-diamino-5'-p-methoxyphenoxycarbonylaminobiphenyl, 2,3'-diamino-6'-p-methoxyphenoxycarbonylaminobiphenyl, 2,4'-diamino-3-p-methoxyphenoxycarbonylaminobiphenyl, 2,4'-diamino-4-p-methoxyphenoxycarbonylaminobiphenyl, 2,4'-diamino-5-p-methoxyphenoxycarbonylaminobiphenyl, 2,4'-diamino-6-p-methoxyphenoxycarbonylaminobiphenyl, 2,4'-diamino-2'-p-methoxyphenoxycarbonylaminobiphenyl, 2,4'-diamino-3'-p-methoxyphenoxycarbonylaminobiphenyl, 3,3'-diamino-2-p-methoxyphenoxycarbonylaminobiphenyl, 3,3'-diamino-4-p-methoxyphenoxycarbonylaminobiphenyl, 3,3'-diamino-5-p-methoxyphenoxycarbonylaminobiphenyl, 3,3'-diamino-6-p-methoxyphenoxycarbonylaminobiphenyl, 3,4'-diamino-2-p-methoxyphenoxycarbonylaminobiphenyl, 3,4'-diamino-4-p-methoxyphenoxycarbonylaminobiphenyl, 3,4'-diamino-5-p-methoxyphenoxycarbonylaminobiphenyl, 3,4'-diamino-6-p-methoxyphenoxycarbonylaminobiphenyl, 3,4'-diamino-2'-p-methoxyphenoxycarbonylaminobiphenyl, 3,4'-diamino-3'-p-methoxyphenoxycarbonylaminobiphenyl, 4,4'-diamino-2-p-methoxyphenoxycarbonylaminobiphenyl, 4,4'-diamino-3-p-methoxyphenoxycarbonylaminobiphenyl,
2-amino-3-carbamoylbiphenyl, 2-amino-4-carbamoylbiphenyl, 2-amino-5-carbamoylbiphenyl, 2-amino-6-carbamoylbiphenyl, 2-amino-2'-carbamoylbiphenyl, 2-amino-3'-carbamoylbiphenyl, 2-amino-4'-carbamoylbiphenyl, 3-amino-2-carbamoylbiphenyl, 3-amino-4-carbamoylbiphenyl, 3-amino-5-carbamoylbiphenyl, 3-amino-6-carbamoylbiphenyl, 3-amino-2'-carbamoylbiphenyl, 3-amino-3'-carbamoylbiphenyl, 3-amino-4'-carbamoylbiphenyl, 4-amino-2-carbamoylbiphenyl, 4-amino-3-carbamoylbiphenyl, 4-amino-2'-carbamoylbiphenyl, 4-amino-3'-carbamoylbiphenyl, 4-amino-4'-carbamoylbiphenyl,
2,2'-diamino-3-carbamoylbiphenyl, 2,2'-diamino-4-carbamoylbiphenyl, 2,2'-diamino-5-carbamoylbiphenyl, 2,2'-diamino-6-carbamoylbiphenyl, 2,3'-diamino-2-carbamoylbiphenyl, 2,3'-diamino-3-carbamoylbiphenyl, 2,3'-diamino-4-carbamoylbiphenyl, 2,3'-diamino-5-carbamoylbiphenyl, 2,3'-diamino-6-carbamoylbiphenyl, 2,3'-diamino-2'-carbamoylbiphenyl, 2,3'-diamino-4'-carbamoylbiphenyl, 2,3'-diamino-5'-carbamoylbiphenyl, 2,3'-diamino-6'-carbamoylbiphenyl, 2,4'-diamino-3-carbamoylbiphenyl, 2,4'-diamino-4-carbamoylbiphenyl, 2,4'-diamino-5-carbamoylbiphenyl, 2,4'-diamino-6-carbamoylbiphenyl, 2,4'-diamino-2'-carbamoylbiphenyl, 2,4'-diamino-3'-carbamoylbiphenyl, 3,3'-diamino-2-carbamoylbiphenyl, 3,3'-diamino-4-carbamoylbiphenyl, 3,3'-diamino-5-carbamoylbiphenyl, 3,3'-diamino-6-carbamoylbiphenyl, 3,4'-diamino-2-carbamoylbiphenyl, 3,4'-diamino-4-carbamoylbiphenyl, 3,4'-diamino-5-carbamoylbiphenyl, 3,4'-diamino-6-carbamoylbiphenyl, 3,4'-diamino-2'-carbamoylbiphenyl, 3,4'-diamino-3'-carbamoylbiphenyl, 4,4'-diamino-2-carbamoylbiphenyl, 4,4'-diamino-3-carbamoylbiphenyl,
2-amino-3-hydroxybiphenyl, 2-amino-4-hydroxybiphenyl, 2-amino-5-hydroxybiphenyl, 2-amino-6-hydroxybiphenyl, 2-amino-2'--hydroxybiphenyl, 2-amino-3'--hydroxybiphenyl, 2-amino-4'--hydroxybiphenyl, 3-amino-2-hydroxybiphenyl, 3-amino-4-hydroxybiphenyl, 3-amino-5-hydroxybiphenyl, 3-amino-6-hydroxybiphenyl, 3-amino-2'--hydroxybiphenyl, 3-amino-3'--hydroxybiphenyl, 3-amino-4'--hydroxybiphenyl, 4-amino-2-hydroxybiphenyl, 4-amino-3-hydroxybiphenyl, 4-amino-2'--hydroxybiphenyl, 4-amino-3'--hydroxybiphenyl, 4-amino-4'-hydroxybiphenyl,
2,2'-diamino-3-hydroxybiphenyl, 2,2'-diamino-4-hydroxybiphenyl, 2,2'-diamino-5-hydroxybiphenyl, 2,2'-diamino-6-hydroxybiphenyl, 2,3'-diamino-2-hydroxybiphenyl, 2,3'-diamino-3-hydroxybiphenyl, 2,3'-diamino-4-hydroxybiphenyl, 2,3'-diamino-5-hydroxybiphenyl, 2,3'-diamino-6-hydroxybiphenyl, 2,3'-diamino-2'--hydroxybiphenyl, 2,3'-diamino-4'-hydroxybiphenyl, 2,3'-diamino-5'--hydroxybiphenyl, 2,3'-diamino-6'--hydroxybiphenyl, 2,4'-diamino-3-hydroxybiphenyl, 2,4'-diamino-4-hydroxybiphenyl, 2,4'-diamino-5-hydroxybiphenyl, 2,4'-diamino-6-hydroxybiphenyl, 2,4'-diamino-2'--hydroxybiphenyl, 2,4'-diamino-3'--hydroxybiphenyl, 3,3'-diamino-2-hydroxybiphenyl, 3,3'-diamino-4-hydroxybiphenyl, 3,3'-diamino-5-hydroxybiphenyl, 3,3'-diamino-6-hydroxybiphenyl, 3,4'-diamino-2-hydroxybiphenyl, 3,4'-diamino-4-hydroxybiphenyl, 3,4'-diamino-5-hydroxybiphenyl, 3,4'-diamino-6-hydroxybiphenyl, 3,4'-diamino-2'--hydroxybiphenyl, 3,4'-diamino-3'-hydroxybiphenyl, 4,4'-diamino-2-hydroxybiphenyl, 4,4'-diamino-3-hydroxybiphenyl,
2-amino-3-hydroxymethylbiphenyl, 2-amino-4-hydroxymethylbiphenyl, 2-amino-5-hydroxymethylbiphenyl, 2-amino-6-hydroxymethylbiphenyl, 2-amino-2'-hydroxymethylbiphenyl, 2-amino-3'-hydroxymethylbiphenyl, 2-amino-4'-hydroxymethylbiphenyl, 3-amino-2-hydroxymethylbiphenyl, 3-amino-4-hydroxymethylbiphenyl, 3-amino-5-hydroxymethylbiphenyl, 3-amino-6-hydroxymethylbiphenyl, 3-amino-2'--hydroxymethylbiphenyl, 3-amino-3'-hydroxymethylbiphenyl, 3-amino-4'-hydroxymethylbiphenyl, 4-amino-2-hydroxymethylbiphenyl, 4-amino-3-hydroxymethylbiphenyl, 4-amino-2'-hydroxymethylbiphenyl, 4-amino-3'-hydroxymethylbiphenyl, 4-amino-4'-hydroxymethylbiphenyl,
2,2'-diamino-3-hydroxymethylbiphenyl, 2,2'-diamino-4-hydroxymethylbiphenyl, 2,2'-diamino-5-hydroxymethylbiphenyl, 2,2'-diamino-6-hydroxymethylbiphenyl, 2,3'-diamino-2-hydroxymethylbiphenyl, 2,3'-diamino-3-hydroxymethylbiphenyl, 2,3'-diamino-4-hydroxymethylbiphenyl, 2,3'-diamino-5-hydroxymethylbiphenyl, 2,3'-diamino-6-hydroxymethylbiphenyl, 2,3'-diamino-2'-hydroxymethylbiphenyl, 2,3'-diamino-4'hydroxymethylbiphenyl, 2,3'-diamino-5'hydroxymethylbiphenyl, 2,3'-diamino-6'hydroxymethylbiphenyl, 2,4'-diamino-3-hydroxymethylbiphenyl, 2,4'-diamino-4-hydroxymethylbiphenyl, 2,4'-diamino-5-hydroxymethylbiphenyl, 2,4'-diamino-6-hydroxymethylbiphenyl, 2,4'-diamino-2'hydroxymethylbiphenyl, 2,4'-diamino-3'hydroxymethylbiphenyl, 3,3'-diamino-2-hydroxymethylbiphenyl, 3,3'-diamino-4-hydroxymethylbiphenyl, 3,3'-diamino-5-hydroxymethylbiphenyl, 3,3'-diamino-6-hydroxymethylbiphenyl, 3,4'-diamino-2-hydroxymethylbiphenyl, 3,4'-diamino-4-hydroxymethylbiphenyl, 3,4'-diamino-5-hydroxymethylbiphenyl, 3,4'-diamino-6-hydroxymethylbiphenyl, 3,4'-diamino-2'hydroxymethylbiphenyl, 3,4'-diamino-3'hydroxymethylbiphenyl, 4,4'-diamino-2-hydroxymethylbiphenyl, 4,4'-diamino-3-hydroxymethylbiphenyl,
2-amino-3-aminomethylbiphenyl, 2-amino-4-aminomethylbiphenyl, 2-amino-5-aminomethylbiphenyl, 2-amino-6-aminomethylbiphenyl, 2-amino-2'-aminomethylbiphenyl, 2-amino-3'-aminomethylbiphenyl, 2-amino-4'-aminomethylbiphenyl, 3-amino-2-aminomethylbiphenyl, 3-amino-4-aminomethylbiphenyl, 3-amino-5-aminomethylbiphenyl, 3-amino-6-aminomethylbiphenyl, 3-amino-2'-aminomethylbiphenyl, 3-amino-3'-aminomethylbiphenyl, 3-amino-4'-aminomethylbiphenyl, 4-amino-2-aminomethylbiphenyl, 4-amino-3-aminomethylbiphenyl, 4-amino-2'-aminomethylbiphenyl, 4-amino-3'-aminomethylbiphenyl, 4-amino-4'-aminomethylbiphenyl,
2,2'-diamino-3-aminomethylbiphenyl, 2,2'-diamino-4-aminomethylbiphenyl, 2,2'-diamino-5-aminomethylbiphenyl, 2,2'-diamino-6-aminomethylbiphenyl, 2,3'-diamino-2-aminomethylbiphenyl, 2,3'-diamino-3-aminomethylbiphenyl, 2,3'-diamino-4-aminomethylbiphenyl, 2,3'-diamino-5-aminomethylbiphenyl, 2,3'-diamino-6-aminomethylbiphenyl, 2,3'-diamino-2'-aminomethylbiphenyl, 2,3'-diamino-4'-aminomethylbiphenyl, 2,3'-diamino-5'-aminomethylbiphenyl, 2,3'-diamino-6'-aminomethylbiphenyl, 2,4'-diamino-3-aminomethylbiphenyl, 2,4'-diamino-4-aminomethylbiphenyl, 2,4'-diamino-5-aminomethylbiphenyl, 2,4'-diamino-6-aminomethylbiphenyl, 2,4'-diamino-2'-aminomethylbiphenyl, 2,4'-diamino-3'-aminomethylbiphenyl, 3,3'-diamino-2-aminomethylbiphenyl, 3,3'-diamino-4-aminomethylbiphenyl, 3,3'-diamino-5-aminomethylbiphenyl, 3,3'-diamino-6-aminomethylbiphenyl, 3,4'-diamino-2-aminomethylbiphenyl, 3,4'-diamino-4-aminomethylbiphenyl, 3,4'-diamino-5-aminomethylbiphenyl, 3,4'-diamino-6-aminomethylbiphenyl, 3,4'-diamino-2'-aminomethylbiphenyl, 3,4'-diamino-3'-aminomethylbiphenyl, 4,4'-di.amino-2-aminomethylbiphenyl, 4,4'-diamino-3-aminomethylbiphenyl, 2-aminomethylbiphenyl, 3-aminomethylbiphenyl, 4-aminomethylbiphenyl, 2,2'-diaminomethylbiphenyl, 2,3'-diaminomethylbiphenyl, 2,4'-diaminomethylbiphenyl, 3,3'-diaminomethylbiphenyl, 3,4'-diaminomethylbiphenyl, 4,4'-diaminomethylbiphenyl,
2-aminomethyl-3-methylbiphenyl, 2-aminomethyl-4-methylbiphenyl, 2-aminomethyl-5-methylbiphenyl, 2-aminomethyl-6-methylbiphenyl, 2-aminomethyl-2'-methylbiphenyl, 2-aminomethyl-3'-methylbiphenyl, 2-aminomethyl-4'-methylbiphenyl, 3-aminomethyl-2-methylbiphenyl, 3-aminomethyl-4-methylbiphenyl, 3-aminomethyl-5-methylbiphenyl, 3-aminomethyl-6-methylbiphenyl, 3-aminomethyl-2'-methylbiphenyl, 3-aminomethyl-3'-methylbiphenyl, 3-aminomethyl-4'-methylbiphenyl, 4-aminomethyl-2-methylbiphenyl, 4-aminomethyl-3-methylbiphenyl, 4-aminomethyl-2'-methylbiphenyl, 4-aminomethyl-3'-methylbiphenyl, 4-aminomethyl-4'-methylbiphenyl,
2,2'-diaminomethyl-3-methylbiphenyl, 2,2'-diaminomethyl-4-methylbiphenyl, 2,2'-diaminomethyl-5-methylbiphenyl, 2,2'-diaminomethyl-6-methylbiphenyl, 2,3'-diaminomethyl-2-methylbiphenyl, 2,3'-diaminomethyl-3-methylbiphenyl, 2,3'-diaminomethyl-4-methylbiphenyl, 2,3'-diaminomethyl-5-methylbiphenyl, 2,3'-diaminomethyl-6-methylbiphenyl, 2,3'-diaminomethyl-2'-methylbiphenyl, 2,3'-diaminomethyl-4'-methylbiphenyl, 2,3'-diaminomethyl-5'-methylbiphenyl, 2,3'-diaminomethyl-6'-methylbiphenyl, 2,4'-diaminomethyl-3-methylbiphenyl, 2,4'-diaminomethyl-4-methylbiphenyl, 2,4'-diaminomethyl-5-methylbiphenyl, 2,4'-diaminomethyl-6-methylbiphenyl, 2,4'-diaminomethyl-2'-methylbiphenyl, 2,4'-diaminomethyl-3'-methylbiphenyl, 3,3'-diaminomethyl-2-methylbiphenyl, 3,3'-diaminomethyl-4-methylbiphenyl, 3,3'-diaminomethyl-5-methylbiphenyl, 3,3'-diaminomethyl-6-methylbiphenyl, 3,4'-diaminomethyl-2-methylbiphenyl, 3,4'-diaminomethyl-4-methylbiphenyl, 3,4'-diaminomethyl-5-methylbiphenyl, 3,4'-diaminomethyl-6-methylbiphenyl, 3,4'-diaminomethyl-2'-methylbiphenyl, 3,4'-diaminomethyl-3'-methylbiphenyl, 4,4'-diaminomethyl-2-methylbiphenyl, 4,4'-diaminomethyl-3-methylbiphenyl,
2-acetyl-3-aminobiphenyl, 2-acetyl4-aminobiphenyl, 2-acetyl5-aminobiphenyl, 2-acetyl6-aminobiphenyl, 2-acetyl-2'-aminobiphenyl, 2-acetyl-3'-aminobiphenyl, 2-acetyl4'-aminobiphenyl, 3-acetyl-2-aminobiphenyl, 3-acetyl4-aminobiphenyl, 3-acetyl5-aminobiphenyl, 3-acetyl6-aminobiphenyl, 3-acetyl-2'-aminobiphenyl, 3-acetyl-3'-aminobiphenyl, 3-acetyl4'-aminobiphenyl, 4-acetyl-2-aminobiphenyl, 4-acetyl-3-aminobiphenyl, 4-acetyl-2'-aminobiphenyl, 4-acetyl-3'-aminobiphenyl, 4-acetyl4'-aminobiphenyl,
3-acetyl-2,2'-diaminobiphenyl, 4-acetyl-2,2'-diaminobiphenyl, 5-acetyl-2,2'-diaminobiphenyl, 2-acetyl-6,2'-diaminobiphenyl, 3-acetyl-2,3'-diaminobiphenyl, 4-acetyl-2,3'-diaminobiphenyl, 5-acetyl-2,3'-diaminobiphenyl, 2-acetyl-6,3'-diaminobiphenyl, 2-acetyl-3,2'-diaminobiphenyl, 4-acetyl-3,2'-diaminobiphenyl, 3-acetyl-5,2'-diaminobiphenyl, 2-acetyl-5,2'-diaminobiphenyl, 3-acetyl-2,4'-diaminobiphenyl, 4-acetyl-2,4'-diaminobiphenyl, 5-acetyl-2,4'-diaminobiphenyl, 6-acetyl-2,4'-diaminobiphenyl, 2-acetyl-4,2'-diaminobiphenyl, 3-acetyl-4,2'-diaminobiphenyl, 2-acetyl-3,3'-diaminobiphenyl, 4-acetyl-3,3'-diaminobiphenyl, 3-acetyl-5,3'-diaminobiphenyl, 2-acetyl-5,3'-diaminobiphenyl, 2-acetyl-3,4'-diaminobiphenyl, 4-acetyl-3,4'-diaminobiphenyl, 3-acetyl-5,4'-diaminobiphenyl, 2-acetyl-5,4'-diaminobiphenyl, 2-acetyl-4,3'-diaminobiphenyl, 3-acetyl-4,3'-diaminobiphenyl, 2-acetyl4,4'-diaminobiphenyl, 3-acetyl-4,4'-diaminobiphenyl,
2-acetoxy-3-aminobiphenyl, 2-acetoxy-4-aminobiphenyl, 2-acetoxy-5-aminobiphenyl, 2-acetoxy-6-aminobiphenyl, 2-acetoxy-2'-aminobiphenyl, 2-acetoxy-3'-aminobiphenyl, 2-acetoxy-4'-aminobiphenyl, 3-acetoxy-2-aminobiphenyl, 3-acetoxy-4-aminobiphenyl, 3-acetoxy-5-aminobiphenyl, 3-acetoxy-6-aminobiphenyl, 3-acetoxy-2'-aminobiphenyl, 3-acetoxy-3'-aminobiphenyl, 3-acetoxy-4'-aminobiphenyl, 4-acetoxy-2-aminobiphenyl, 4-acetoxy-3-aminobiphenyl, 4-acetoxy-2'-aminobiphenyl, 4-acetoxy-3'-aminobiphenyl, 4-acetoxy-4'-aminobiphenyl,
3-acetoxy-2,2'-diaminobiphenyl, 4-acetoxy-2,2'-diaminobiphenyl, 5-acetoxy-2,2'-diaminobiphenyl, 2-acetoxy-6,2'-diaminobiphenyl, 3-acetoxy-2,3'-diaminobiphenyl, 4-acetoxy-2,3'-diaminobiphenyl, 5-acetoxy-2,3'-diaminobiphenyl, 2-acetoxy-6,3'-diaminobiphenyl, 2-acetoxy-3,2'-diaminobiphenyl, 4-acetoxy-3,2'-diaminobiphenyl, 3-acetoxy-5,2'-diaminobiphenyl, 2-acetoxy-5,2'-diaminobiphenyl, 3-acetoxy-2,4'-diaminobiphenyl, 4-acetoxy-2,4'-diaminobiphenyl, 5-acetoxy-2,4'-diaminobiphenyl, 6-acetoxy-2,4'-diaminobiphenyl, 2-acetoxy-4,2'-diaminobiphenyl, 3-acetoxy-4,2'-diaminobiphenyl, 2-acetoxy-3,3'-diaminobiphenyl, 4-acetoxy-3,3'-diaminobiphenyl, 3-acetoxy-5,3'-diaminobiphenyl, 2-acetoxy-5,3'-diaminobiphenyl, 2-acetoxy-3,4'-diaminobiphenyl, 4-acetoxy-3,4'-diaminobiphenyl, 3-acetoxy-5,4'-diaminobiphenyl, 2-acetoxy-5,4'-diaminobiphenyl, 2-acetoxy-4,3'-diaminobiphenyl, 3-acetoxy-4,3'-diaminobiphenyl, 2-acetoxy-4,4'-diaminobiphenyl, 3-acetoxy-4,4'-diaminobiphenyl,
2-acetylthioxy-3-aminobiphenyl, 2-acetylthioxy-4-aminobiphenyl, 2-acetylthioxy-5-aminobiphenyl, 2-acetylthioxy-6-aminobiphenyl, 2-acetylthioxy-2'-aminobiphenyl, 2-acetylthioxy-3'-aminobiphenyl, 2-acetylthioxy-4'-aminobiphenyl, 3-acetylthioxy-2-aminobiphenyl, 3-acetylthioxy-4-aminobiphenyl, 3-acetylthioxy-5-aminobiphenyl, 3-acetylthioxy-6-aminobiphenyl, 3-acetylthioxy-2'-aminobiphenyl, 3-acetylthioxy3'-aminobiphenyl, 3-acetylthioxy-4',4-acetylthioxy-2'-aminobiphenyl, 4-acetylthioxy-3'-aminobiphenyl, 4-acetylthioxy-4'-aminobiphenyl,
3-acetylthioxy-2,2'-diaminobiphenyl, 4-acetylthioxy-2,2'-diaminobiphenyl, 5-acetylthioxy-2,2'-diaminobiphenyl, 2-acetylthioxy6,2'-diaminobiphenyl, 3-acetylthioxy-2,3'-diaminobiphenyl, 4-acetylthioxy-2,3'-diaminobiphenyl, 5-acetylthioxy-2,3'-diaminobiphenyl, 2-acetylthioxy-6,3'-diaminobiphenyl, 2-acetylthioxy-3,2'-diaminobiphenyl, 4-acetylthioxy-3,2'-diaminobiphenyl, 3-acetylthioxy-5,2'-diaminobiphenyl, 2-acetylthioxy-5,2'-diaminobiphenyl, 3-acetylthioxy-2,4'-diaminobiphenyl, 4-acetylthioxy-2,4'-diaminobiphenyl, 5-acetylthioxy-2,4'-diaminobiphenyl, 6-acetylthioxy-2,4'-diaminobiphenyl, 2-acetylthioxy-4,2'-diaminobiphenyl, 3-acetylthioxy-4,2'-diaminobiphenyl, 2'-acetylthioxy-3,3'-diaminobiphenyl, 4-acetylthioxy-3,3'-diaminobiphenyl, 3-acetylthioxy-5,3'-diaminobiphenyl, 2-acetylthioxy-5,3'-diaminobiphenyl, 2-acetylthioxy-3,4'-diaminobiphenyl, 4-acetylthioxy-3,4'-diaminobiphenyl, 3-acetylthioxy-5,4'-diaminobiphenyl, 2-acetylthioxy-5,4'-diaminobiphenyl, 2-acetylthioxy-4,3'-diaminobiphenyl, 3-acetylthioxy-4,3'-diaminobiphenyl, 2-acetylthioxy-4,4'-diaminobiphenyl, 3-acetylthioxy-4,4'-diaminobiphenyl,
2-(2-aminophenyl)-pyridine, 2-(3-aminophenyl)-pyridine, 2-(4-aminophenyl)-pyridine, 3-(2-aminophenyl)-pyridine, 3-(3-aminophenyl)-pyridine, 3-(4-aminophenyl)-pyridine, 4-(2-aminophenyl)-pyridine, 4-(3-aminophenyl)-pyridine, 4-(4-aminophenyl)-pyridine,
2-(2-aminomethylphenyl)-pyridine, 2-(3-aminomethylphenyl)-pyridine, 2-(4-aminomethylphenyl)-pyridine, 3-(2-aminomethylphenyl)-pyridine, 3-(3-aminomethylphenyl)-pyridine, 3-(4-aminomethylphenyl)-pyridine, 4-(2-aminomethylphenyl)-pyridine, 4-(3-aminomethylphenyl)-pyridine, 4-(4-aminomethylphenyl)-pyridine,
2-(2-dimethylaminophenyl)-pyridine, 2-(3-dimethylaminophenyl)-pyridine, 2-(4-dimethylaminophenyl)-pyridine, 3-(2-dimethylaminophenyl)-pyridine, 3-(3-dimethylaminophenyl)-pyridine, 3-(4-dimethylaminophenyl)-pyridine, 4-(2-dimethylaminophenyl)-pyridine, 4-(3-dimethylaminophenyl)-pyridine, 4-(4-dimethylaminophenyl)-pyridine,
2-(2-methylaminophenyl)-pyridine, 2-(3-methylaminophenyl)-pyridine, 2-(4-methylaminophenyl)-pyridine, 3-(2-methylaminophenyl)-pyridine, 3-(3-methylaminophenyl)-pyridine, 3-(4-methylaminophenyl)-pyridine, 4-(2-methylaminophenyl)-pyridine, 4-(3-methylaminophenyl)-pyridine, 4-(4-methylaminophenyl)-pyridine,
2-(2-methylaminophenyl)-pyridine, 2-(3-methylaminophenyl)-pyridine, 2-(4-methylaminophenyl)-pyridine, 3-(2-methylaminophenyl)-pyridine, 3-(3-methylaminophenyl)-pyridine, 3-(4-methylaminophenyl)-pyridine, 4-(2-methylaminophenyl)-pyridine, 4-(3-methylaminophenyl)-pyridine, 4-(4-methylaminophenyl)-pyridine, 3-amino-2-phenylpyridine, 4-amino-2-phenylpyridine, 5-amino-2-phenylpyridine, 6-amino-2-phenylpyridine, 2-amino-3-phenylpyridine, 2-amino-4-phenylpyridine, 2-amino-5-phenylpyridine, 2-amino-6-phenylpyridine, 3-amino-4-phenylpyridine, 3-amino-5-phenylpyridine, 4-amino-3-phenylpyridine,
2-(2-aminophenyl)-furyl, 2-(3-aminophenyl)-furyl, 2-(4-aminophenyl)-furyl, 3-(2-aminophenyl)-furyl, 3-(3-aminophenyl)-furyl, 3-(4-aminophenyl)-furyl,
2-(2-aminophenyl)-thienyl, 2-(3-aminophenyl)-thienyl, 2-(4-aminophenyl)-thienyl, 3-(2-aminophenyl)-thienyl, 3-(3-aminophenyl)-thienyl, 3-(4-aminophenyl)-thienyl,
2-(2-aminomethylphenyl)-furyl, 2-(3-aminomethylphenyl)-furyl, 2-(4-aminomethylphenyl)-furyl, 3-(2-aminomethylphenyl)-furyl, 3-(3-aminomethylphenyl)-furyl, 3-(4-aminomethylphenyl)-furyl,
2-(2-aminomethylphenyl)-thienyl, 2-(3-aminomethylphenyl)-thienyl, 2-(4-aminomethylphenyl)-thienyl, 3-(2-aminomethylphenyl)-thienyl, 3-(3-aminomethylphenyl)-thienyl, 3-(4-aminomethylphenyl)-thienyl, and the like.

According to the present invention, biaryl compounds having an amino group can be produced simply and efficiently.

### EXAMPLES

The present invention will be illustrated further in detail below, but it is needless to say the the present invention is not limited to these examples. The yield in the examples means a yield after isolation.

### Example 1

209 mg (1.71, mmol) of phenylboronic acid, 168 mg (1.32 mmol) of 2-chloroaniline, 18 mg (0.065 mmol) of bis(1,5-cyclooctadiene)nickel, 37 mg (0.13 mmol) of tricyclohexyl phosphine, 1.00 g (4.71 mmol) of tri-potassium phosphate, 31 mg (1.71 mmol) of distilled water and 3 ml of 1,4-dioxane were mixed. Then, the mixture was heated up to 85 °C, then, stirred for 4 hours at the same temperature. After completion of the reaction, the mixture was left to cool to room temperature, and the resulting reaction mixture was subjected to a filtration treatment. The filtrate was concentrated under reduced pressure, then, the resultant residue was purified by silica gel column chromatography, to obtain 2-aminobiphenyl in a yield of 81% (based on 2-chloroaniline).

### Example 2

Operations were carried out according to Example 1 excepting that 168 mg (1.32 mmol) of 3-chloroaniline was used instead of 2-chloroaniline. 3-aminobiphenyl was obtained in a yield of 78% (based on 3-chloroaniline), instead of 2-aminobiphenyl.

### Example 3

Operations were carried out according to Example 1 excepting that 187 mg (1.32 mmol) of 4-chlorobenzylamine was used instead of 2-chloroaniline. 4-phenylbenzylamine was obtained in a yield of 74% (based on 4-chlorobenzylamine), instead of 2-aminobiphenyl.

### Example 4

326 mg (1.71, mmol) of 4-(trifluoromethyl)phenylboronic acid, 168 mg (1.32 mmol) of 2-chloroaniline, 18 mg (0.065 mmol) of bis(1,5-cyclooctadiene)nickel, 37 mg (0.13 mmol) of tricyclohexyl phosphine, 1.00 g (4.71 mmol) of tri-potassium phosphate, 31 mg (1.71 mmol) of distilled water and 3 ml of 1,4-dioxane were mixed. Then, the mixture was heated up to 85 °C, then, stirred for 4 hours at the same temperature. After completion of the reaction, the mixture was left to cool to room temperature, and the resulting reaction mixture was subjected to a filtration treatment. The filtrate was concentrated under reduced pressure, then, the resultant residue was purified by silica gel column chromatography, to obtain 2-amino-4'-trifluoromethyl-1,1'-biphenyl in a yield of 76% (based on 2-chloroaniline).

### Example 5

Operations were carried out according to Example 4 excepting that 168 mg (1.32 mmol) of 3-chloroaniline was used instead of 2-chloroaniline. 3-amino-4'-trifluoromethyl-1,1'-biphenyl was obtained in a yield of 82% (based on 3-chloroaniline), instead of 2-amino-4'-trifluoromethyl-1,1'-biphenyl.

### Example 6

Operations were carried out according to Example 4 excepting that 168 mg (1.32 mmol) of 4-chloroaniline was used instead of 2-chloroaniline. 4-amino-4'-trifluoromethyl-1,1'-biphenyl was obtained in a yield of 71% (based on 4-chloroaniline), instead of 2-amino-4'-trifluoromethyl-1,1'-biphenyl.

### Example 7

Operations were carried out according to Example 4 excepting that 187 mg (1.32 mmol) of 4-chlorobenzylamine was used instead of 2-chloroaniline. 4-aminomethyl-4'-trifluoromethyl-1,1'-biphenyl was obtained in a yield of 75% (based on 4-chlorobenzylamine), instead of 2-amino-4'-trifluoromethyl-1,1'-biphenyl.

### Example 8

319 mg (1.71, mmol) of 3-aminophenylboronic acid·hemi-sulfate, 167 mg (1.32 mmol) of 4-chlorotoluene, 18 mg (0.065 mmol) of bis(1,5-cyclooctadiene)nickel, 37 mg (0.13 mmol) of tricyclohexyl phosphine, 1.00 g (4.71 mmol) of tri-potassium phosphate, 31 mg (1.71 mmol) of distilled water and 3 ml of 1,4-dioxane were mixed. Then, the mixture was heated up to 85 °C, then, stirred for 4 hours at the same temperature. After completion of the reaction, the mixture was left to cool to room temperature, and subjected to a filtration treatment. The filtrate was concentrated under reduced pressure, then, the resultant residue was purified by silica gel column chromatography, to obtain 3-amino-4'-methyl-1,1'-biphenyl in a yield of 87% (based on 4-chlorotoluene).

### Example 9

Operations were carried out according to Example 8 excepting that 204 mg (1.32 mmol) of 4-chloroacetophenone was used instead of 4-chlorotoluene. 3-amino-4'-acetyl-1,1'-biphenyl was obtained in a yield of 100% (based on 4-chloroacetophene), instead of 3-amino-4'-methyl-1,1'-biphenyl.

### Example 10 '

Operations were carried out according to Example 8 excepting that 204 mg (1.32 mmol) of 4-chloroacetophenone was used instead of 4-chlorotoluene and the reaction temperature was 60 °C. 3-amino-4'-acetyl-1,1'-biphenyl was obtained in a yield of 100% (based on 4-chloroacetophene), instead of 3-amino-4'-methyl-1,1'-biphenyl.

### Comparative Example 1

319 mg (1.71, mmol) of 3-aminophenylboronic acid·hemi-sulfate, 204 mg (1.32 mmol) of 4-chloroacetophenone, 43 mg (0.065 mmol) of dichlorobistrisphenyl phosphine nickel (II), 1.00 g (4.71 mmol) of tri-potassium phosphate, 31 mg (1.71 mmol) of distilled water and 3 ml of toluene were mixed. Then, the mixture was heated up to 85 °C, then, stirred for 4 hours at the same temperature. After completion of the reaction, the mixture was left to cool to room temperature, and subjected to a filtration treatment. The filtrate was analyzed in its content to find that the yield of 3-amino-4'-acetyl-1,1'-biphenyl was 0.2% (based on 4-chloroacetophenone).

### INDUSTRIAL APPLICABILITY

The biaryl compound having an amino group obtained by the production method of the present invention can be used suitably as a chemical raw material, or an intermediate of medical and agricultural drugs, and the like.

## Claims

1. A method for producing a biaryl compound of the following formula (4), comprising reacting an aromatic organic compound of the following formula (1) with at least one compound selected from the group consisting of aromatic organoboron compounds of the following formula (2) and boroxine compounds of the following formula (3), in the presence of a zero-valent nickel catalyst, phosphine ligand and base: 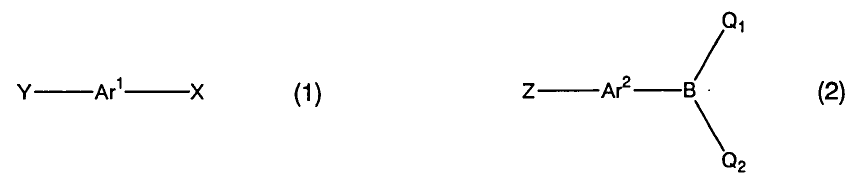 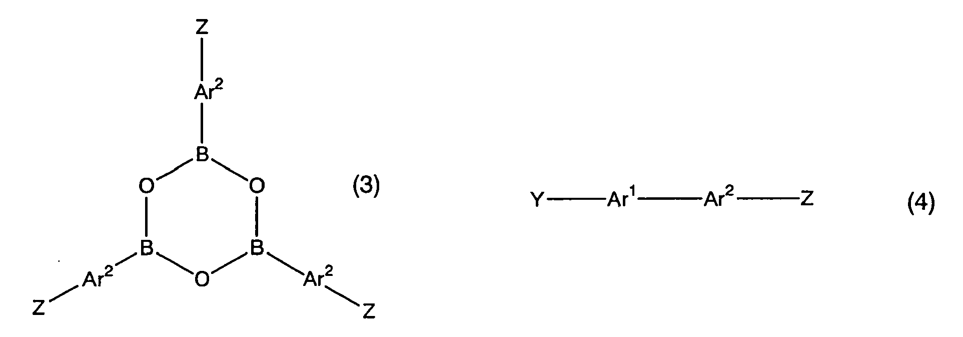 (wherein, Ar¹ and Ar² represent each independently an arylene group or heteroarylene group, the arylene group or heteroarylene group may have at least one substituent selected from the group consisting of optionally substituted alkyl groups, optionally substituted cycloalkyl groups, optionally substituted aryl groups, optionally substituted heteroaryl groups, optionally substituted alkoxy groups, optionally substituted aryloxy groups, optionally substituted alkylthio groups, optionally substituted arylthio groups, optionally substituted alkylcarbonyl groups, optionally substituted arylcarbonyl groups, optionally substituted alkylamino groups, optionally substituted alkylcarbonylamino groups, optionally substituted alkoxycarbonylamino groups, optionally substituted alkanesulfoneamide groups, optionally substituted arenesulfoneamide groups, optionally substituted N-arylcarbamoyl groups, optionally substituted imide groups, fluorine atom, hydroxyl group, formyl group, amino group, carbamoyl group, sulfamoyl group, mercapto group and imino group, substituents on adjacent carbon atoms on the arylene group or heteroarylene group may be mutually connected to form a condensed ring together with the arylene group or heteroarylene group,
Q₁ and Q₂ represent each independently a hydroxyl group or an alkoxy group having a carbon number of 1 to 4, or Q₁ and Q₂ are connected to represent an alkylenedioxy group having a carbon number of 1 to 6 or a catecholate group,
X represents a halogen atom, alkylsulfonyloxy group or arylsulfonyloxy group,
Y and Z represent each independently a hydrogen atom, amino group or aminoalkyl group, Y and Z do not simultaneously represent a hydrogen atom, and a hydrogen atom in the alkyl of said aminoalkyl group may be substituted by a carboxyl group or alkoxycarbonyl group.).

2. The production method according to Claim 1, wherein the reaction is carried out in the presence of an ether solvent.

3. The production method according to Claim 1 or 2, wherein the zero-valent nickel catalyst is tetrakis(triphenyl phosphine)nickel(0), tetracarbonylnickel(0) or bis(1,5-cyclooctadiene)nickel(0).

4. The production method according to any one of Claims 1 to 3, wherein the zero-valent nickel catalyst is bis(1,5-cycloctadiene)nickel(0).

5. The production method according to any one of Claims 1 to 4, wherein the phosphine ligand is a trialkyl phosphine, tricycloalkyl phosphine or triaryl phosphine.

6. The production method according to any one of Claims 1 to 5, wherein the phosphine ligand is a tricycloalkyl phosphine.

7. The production method according to any one of Claims 1 to 6, wherein the base is at least one selected from the group consisting of alkali metal hydroxides, alkaline earth metal hydroxides, alkali metal carbonates, alkaline earth metal carbonates, alkali metal carboxylates, alkaline earth metal carboxylates, alkali metal hydrogencarbonates, alkaline earth metal hydrogencarbonates, alkali metal phosphates, alkali metal fluorides and tertiary amines, or its hydrate.

8. The production method according to any one of Claims 1 to 7, wherein the base is an alkali metal phosphates.

9. The production method according to any one of Claims 1 to 8, wherein X in the formula (1) is a halogen atom.

10. The production method according to Claim 9, wherein the halogen atoms is a chlorine atom.
